# EUROPEAN PATENT SPECIFICATION

(11) **EP 3 339 437 B1**
(45) Date of publication and mention of the grant of the patent: **17.11.2021**
(21) Application number: 16836825.6
(22) Date of filing: 23.03.2016
(51) Int. Cl.: C12N 15/09, C12N 1/15, C12N 1/19, C12N 5/10

(54) **METHOD FOR SITE-SPECIFIC INSERTION OF FOREIGN DNA INTO ANIMAL CELL GENOME AND CELL OBTAINED USING SAME**
VERFAHREN ZUR ORTSSPEZIFISCHEN EINFÜHRUNG VON FREMDER DNS IN TIERISCHES ZELLGENOM UND HERGESTELLTE ZELLE DAMIT
PROCÉDÉ D'INSERTION SPÉCIFIQUE DE SITE D'UN ADN ÉTRANGER DANS LE GÉNOME D'UNE CELLULE ANIMALE ET CELLULE AINSI OBTENUE

(30) Priority: 20.08.2015 JP 2015162612; 10.12.2015 US 201562265425 P
(43) Date of publication of application: 27.06.2018
(73) Proprietor: Inter-University Research Institute Corporation Research Organization of Information and Systems, Tokyo 190-8562 (JP)
(72) Inventor: KANEMAKI, Masato, Mishima-shi Shizuoka 411-8540 (JP); NATSUME, Toyoaki, Mishima-shi Shizuoka 411-8540 (JP)
(74) Representative: HGF
(86) International application number: PCT/JP2016/059174
(87) International publication number: WO 2017/029833

(56) References cited:
- WO-A1-2010/125620
- JP-A- 2010 524 500
- JP-A- 2011 518 555
- JP-A- 2015 523 384
- US-A1- 2013 203 840
- US-A1- 2015 128 300
- SILVIA PETREZSELYOVA ET AL: "Homology arms of targeting vectors for gene insertions and CRISPR/Cas9 technology: size does not matter; quality control of targeted clones does.", CELLULAR AND MOLECULAR BIOLOGY LETTERS, vol. 20, no. 5, 23 November 2015 (2015-11-23), XP055399688, DOI: 10.1515/cmble-2015-0047
- Y. PENG ET AL: "Making designer mutants in model organisms", DEVELOPMENT, vol. 141, no. 21, 21 October 2014 (2014-10-21), pages 4042-4054, XP055467125, GB ISSN: 0950-1991, DOI: 10.1242/dev.102186
- LEE, JAE SEONG ET AL.: 'Site-specific integration in CHO cells mediated by CRISPR/Cas9 and homology-directed DNA repair pathway' SCI. REP. vol. 5, no. 8572, 25 February 2015, pages 1 - 11, XP055373118
- LI, KAI ET AL.: 'Optimization of genome engineering approaches with the CRISPR/Cas9 system' PLOS ONE vol. 9, 2004, pages 1 - 10, XP055243646
- NISHIMURA, KOHEI ET AL.: 'Rapid Depletion of Budding Yeast Proteins via the Fusion of an Auxin-Inducible Degron (AID' CURR. PROTOC. CELL BIOL. 2014, XP055373122
- KUBOTA, TAKASHI ET AL.: 'The Elgl replication factor C-like complex functions in PCNA unloading during DNA replication' MOL. CELL vol. 50, 2013, pages 273 - 280, XP028589789
- RONDA, CARLOTTA ET AL.: 'CrEdit: CRISPR mediated multi-loci gene integration in Saccharomyces cerevisiae' MICROB. CELL FACT. vol. 14, no. 97, 07 July 2015, pages 1 - 11, XP021226447
- LUO, YONGQUAN ET AL.: 'Stable enhanced green fluorescent protein expression after differentiation and transplantation of reporter human induced pluripotent stem cells generated by AAVS1 transcription activator-like effector nucleases' STEM CELLS TRANSL. MED. vol. 3, 2014, pages 821 - 835, XP055373126
- ZOU, JIZHONG ET AL.: 'Oxidase-deficient neutrophils from X-linked chronic granulomatous disease iPS cells: functional correction by zinc finger nuclease-mediated safe harbor targeting' BLOOD vol. 117, 2011, pages 5561 - 5572, XP055040721
- NISHIMURA, KOHEI ET AL.: 'An auxin-based degron system for the rapid depletion of proteins in nonplant cells' NAT. METHODS. vol. 6, 2009, pages 917 - 922, XP055373128
- NATSUME, TOYOAKI ET AL.: 'Rapid Protein Depletion in Human Cells by Auxin-Inducible Degron Tagging with Short Homology Donors' CELL REP. vol. 15, 05 April 2016, pages 210 - 218, XP055373129

## Description

### Technical Field

The present invention relates to an in vitro method for site-specific insertion of foreign DNA into a genome in an animal cell.

Priority is claimed on Japanese Patent Application No. 2015-162612, filed on August 20, 2015, and United States Provisional Patent Application No. 62/265425, filed on December 10, 2015.

### Background Art

In recent years, a technology for introducing site-specific modification into a genome in an animal cell, a so-called genome-editing technology, has advanced dramatically. That is, with the availability of genome-editing technology such as Zn finger nuclease (ZFN), TALEN, or CRISPR-Cas9, it has recently become possible to insert foreign DNA into a specific genomic site in cultured cells using homology direct repair (HDR) after DNA cleavage. By using this technology, a tag such as GFP can be added to an endogenous gene (NPL 1 to 7). Meanwhile, a method of modifying a site-specific sequence by configuring the single-stranded oligo DNA to have a homologous region of 50 to 80 b has also been developed (NPL 7 and 8). With respect to fly S2 cells, a foreign DNA insertion method using DNA fragments amplified by PCR using homology-added primers as they are has been developed (NPL 9). On the other hand, a technology for adding a tag using a micro-homology end-joining (MMEJ) pathway which is another repair pathway, without using the HDR pathway after genome cleavage by genome editing has also been developed (NPL 10).

### Citation List

### Non-Patent Literature

[NPL 1]: Nat. Biotech., vol. 27, P851-857 (2009)
[NPL 2]: Nat. Biotech., vol. 29, P731-734 (2011)
[NPL 3]: Science, vol. 339, P823-825 (2013)
[NPL 4]: Nat. Biotech., vol. 29, P143-148 (2011)
[NPL 5]: PLoS One, DOI: 10.1371/journal. pone.0095101 (2014)
[NPL 6]: Sci Reports, DOI: 10.1038/srep09592 (2015)
[NPL 7]: Nat. Protocols, Vol. 8 No. 11, P2281-2307 (2013)
[NPL 8]: Cell, vol. 153, P910-918 (2013)
[NPL 9]: Nuc. Acid. Res., Vol. 42, e89, DOI: 10.1093/nar/gku289 (2014)
[NPL 10]: Nat. Commun., DOI: 10.1038/ncomms6560 (2014)

Lee et al, "Site-specific integration in CHO cells mediated by CRISPR/Cas9 and homology-directed DNA repair pathway, Sci. Rep., 2015.02.25, Vol 5: 8572, pp1-11, reports efficient targeted gene integration into site-specific loci in CHO cells using a CRISPR/Cas9 genome editing system. Li et al, "Optimization of genome engineering approaches with the CRISPR/Cas9 system" PLoS One, 2004, Vol. 9: e105779, pp1-10, explores several parameters that influence Cas9-mediated scarless genome editing efficiency in murine embryonic stem cells. US2013/203840 A1 reports the use of meganucleases for inducing homologous recombination ex vivo and in toto in vertebrate somatic tissues, and the application thereof. US2015/128300 A1 reports methods and compositions for generating conditional knock-out alleles.

### Summary of Invention

### Technical Problem

However, in the case of a method using homology-directed repair (HDR), for a plasmid (donor plasmid) that serves as a template for HDR, it is necessary to clone, from the genomic DNA, a sequence of about 0.7 kbp or more each on the right and left (1.4 kbp or more in total) homologous to an insertion site on a genome (NPL 1 to 7). Therefore, there was a problem in that complicated cloning in accordance with the intended use is required. On the other hand, in the case of a method using single-stranded oligo DNA, it is merely possible to insert a foreign sequence of about 1 to 20 bp and therefore such a method could not be used for adding a tag such as GFP.

Therefore, there is a need in the art for a genome-editing technology capable of inserting long foreign DNA of 0.1 kbp or more into the genome by a simple operation that does not require a cloning operation of genomic DNA.

### Solution to Problem

Accordingly, since it has been thought that cloning of a homologous sequence of 0.7 kbp or more each on the right and left on the genome is indispensable for insertion of foreign DNA by an HDR pathway using a genome-editing technology such as a CRISPR-Cas9 system (NPL 1 to 7), the present inventors have constructed a donor plasmid by designing such a homologous sequence to a sequence that is short enough to be obtained by PCR or DNA synthesis. As a result, the present inventors unexpectedly have found that foreign DNA of 0.1 kbp to 10 kbp can be inserted into a target site on a genome with sufficient efficiency by HDR using a donor plasmid in which large foreign DNA of 0.1 kbp to 10 kbp is ligated between short homologous sequences of 125 bp to 200 bp which are synthesized by PCR or DNA synthesis. The present invention has been completed based on these findings.

That is, the present invention provides.
[1] An in vitro method for site-specific insertion of foreign DNA into a genome in an animal cell, including:
   (1) a step of constructing a donor plasmid in which DNA of 125 bp to 200 bp having a sequence homologous to an insertion site on a genome is ligated to the upstream and downstream of foreign DNA of 0.1 kbp to 10 kbp; and
   (2) a step of inserting the foreign DNA of 0.1 kbp to 10 kbp into a target site on the genome by homology-directed repair-based (HDR-based) genome editing using the obtained donor plasmid, wherein the animal cell is a human HCT116 cell, or a mouse ES cell,
      wherein the homology-directed repair-based genome editing method is a foreign DNA insertion method using a CRISPR-Cas9 system,
      and wherein the DNA having a sequence homologous to the inserted genomic site is obtained by PCR or DNA synthesis.
[2] The in vitro method for site-specific insertion of foreign DNA into a genome according to [1], in which the foreign DNA is foreign DNA selected from tagged foreign DNA, a promoter sequence, a transcription termination sequence, a functional gene sequence, a drug selection marker gene, and a combination thereof.

### Advantageous Effects of Invention

According to the method of the present invention using a donor plasmid, since the length of a sequence homologous to the genomic DNA necessary for HDR using genome editing is as short as 125 bp to 200 bp, there is no need for cloning such a homologous sequence, and such a homologous sequence can be conveniently constructed by PCR or DNA synthesis and is therefore comprehensively applicable to numerous genes and target genomic sites. In addition, a large sequence such as a fluorescent tag, a purification tag, a degron tag, or a drug selection marker may be inserted as the foreign DNA capable of being inserted. In addition, by inserting foreign DNA into any gene by the method of the present invention, it is possible to obtain a cell that constitutively expresses the foreign DNA. For example, according to the cell obtained by the method of the present invention and including a chromosome containing a first gene encoding a target protein and a gene encoding mAID ligated to the downstream of the first gene, and a chromosome containing a gene encoding transport inhibitor response 1 (TIR1) at a safe harbor locus, the degradation of the target protein can be induced efficiently.

### Brief Description of Drawings

FIG. 1 shows an example of a strategy of donor plasmid construction according to the method of the present disclosure using a PCR method or DNA synthesis.
FIG. 2 shows a strategy for adding GFP to an MCM8 gene.
FIG. 3 shows a genomic structure before and after insertion of foreign DNAs (GFP and neomycin resistance selection marker) used in Example 1 and confirmation of foreign DNA insertion by PCR.
FIG. 4 shows the results of Western blotting indicating that an MCM8 protein with GFP added thereto is expressed in foreign DNA-introduced cells.
FIG. 5 shows a strategy for adding mAID-Clover to a RAD21 gene.
FIG. 6 shows an example in which a mAID-Clover tag (truncated auxin-induced degron+fluorescent tag) is introduced into a RAD21 gene.
FIG. 7 shows that a treatment with auxin induces the degradation of RAD21-mAID-Clover which in turn disappears from a nucleus.
FIG. 8 shows a strategy for adding mAID-Clover to a DHC1 gene.
FIG. 9 shows an example in which a mAID-Clover tag (truncated auxin-induced degron+fluorescent tag) is introduced into a DHC1 gene.
FIG. 10 shows an example in which a mAID-Clover tag (truncated auxin-induced degron+fluorescent tag) is introduced into a DHC1 gene.
FIG. 11 is a graph showing the relative number of cells at 24, 48, and 72 hours after treatment with respect to the number of cells at zero hours after treatment, using living cells in which a DHC1-mAID-Clover fusion protein is expressed, untreated and treated with 2 µg/mL of doxycycline alone, or 2 µg/mL of doxycycline and 500 µM of auxin.
FIG. 12 is a graph showing the percentage of mitotic cells at 18 and 42 hours after treatment, using living cells in which a DHC1-mAID-Clover fusion protein is expressed, untreated and treated with 1 µg/mL of tetracycline alone, or 1 µg/mL of tetracycline and 500 µM of auxin.
FIG. 13 shows the results of fluorescence microscope observation after immunostaining with a tubulin antibody and nuclear staining with SiR-Hoechst at 18 hours after treatment for living cells in which a DHC1-mAID-Clover fusion protein is expressed, untreated and treated with 1 µg/mL of tetracycline and 500 µM of auxin.
FIG. 14 shows a strategy for adding mAID-Clover to an MCM2 gene of a mouse ES cell.
FIG. 15 shows the results of observation of a mouse ES cell in which an MCM2-mAID-Clover fusion protein is expressed under a deconvolution fluorescence microscope.

### Description

### <<Method for site-specific insertion of foreign DNA into a genome in an animal cell >>

Described herein is a method for site-specific insertion of foreign DNA into a genome in an animal cell which includes the following steps (1) and (2):
(1) a step of constructing a donor plasmid in which DNA of 100 bp to 300 bp having a sequence homologous to an insertion site on a genome is ligated to the upstream and downstream of foreign DNA of 0.1 kbp to 10 kbp; and
(2) a step of inserting the foreign DNA of 0.1 kbp to 10 kbp into a target site on the genome by HDR-based genome editing using the obtained donor plasmid.

In the method, not according to the claimed invention and present for the illustration purpose only, the animal cell targeted for foreign DNA insertion may be an established animal-derived cell, ES cell, or iPS cell, and examples thereof include cells selected from an established human-derived cell, an established mouse-derived cell, an established chicken-derived cell, a human ES cell, a mouse ES cell, a human iPS cell, and a mouse iPS cell. Among those cells, from the viewpoint that HDR-based genome editing will proceed efficiently even in the case where the sequence homologous to an insertion site on a genome is short DNA of 100 bp to 300 bp, a cell may be selected from a human HCT116 cell, a human HT1080 cell, a human NALM6 cell, a human ES cell, a human iPS cell, a mouse ES cell, a mouse iPS cell, and a chicken DT40 cell, or selected from a HCT116 cell, a human ES cell, a human iPS cell, a mouse ES cell, and a mouse iPS cell. In the method of the invention, the animal cell is a human HCT116 cell, or a mouse ES cell.

From the viewpoint of the size of a gene itself to be added at the time of adding a tag, the viewpoint of inserting a functional expression unit, and the viewpoint of adding a drug selection marker gene, the foreign DNA capable of being inserted into the genome is foreign DNA having a length of 0.1 kbp to 10 kbp, for example foreign DNA having a length of 1 kbp to 10 kbp, foreign DNA having a length of 1 kbp to 8 kbp, or foreign DNA having a length of 1 kbp to 5 kbp. Examples of the foreign DNA having such a length include tagged foreign DNA, a promoter sequence, a transcription termination sequence, a functional gene sequence, a drug selection marker gene, and a combination thereof. Examples of the tag include a fluorescent tag (such as GFP), an affinity tag, a degron tag, and a localization tag. In addition, foreign genes (functional genes) having a variety of functions can be introduced downstream of these tags. For example, a drug selection marker gene can be introduced. In the method of the invention, the foreign DNA has a length of 0.1 kbp to 10 kbp.

Examples of the drug selection marker gene for use in the present disclosure include a neomycin-resistant gene, a histidinol-resistant gene, a puromycin-resistant gene, a hygromycin-resistant gene, and a blasticidin drug-resistant gene. A cell into which foreign DNA has been introduced can be selected by inserting a drug selection marker gene and culturing the cell using a medium containing such a drug.

First, in step (1), a donor plasmid in which DNA of 100 bp to 300 bp having a sequence homologous to an insertion site on a genome is ligated to the upstream and downstream of foreign DNA of 0.1 kbp to 10 kbp to be inserted is constructed.

The DNA of 100 bp to 300 bp having a sequence homologous to an insertion site on a genome can be easily obtained by PCR or DNA synthesis, due to having a short length of 100 bp to 300 bp. That is, cloning of a long homologous site of 0.7 kbp or more is not necessary unlike a conventional genome editing method using HDR. The oligo DNA may be 125 bp to 300 bp, 125 bp to 250 bp, 150 bp to 250 bp, or 170 bp to 250 bp. In the method of the invention, the DNA having a sequence homologous to an insertion site on a genome is 125bp to 200bp. The sequence of the DNA itself can be appropriately determined from a database relating to a host animal. In the case where the sequence of the DNA can be determined, the DNA can be synthesized by DNA synthesis or can be constructed by adding a homologous sequence to a synthetic primer for amplifying the DNA, followed by PCR amplification using them. In the method of the invention, the DNA having a sequence homologous to the inserted genomic site is obtained by PCR or DNA synthesis.

The above DNA having a homologous region is ligated to the upstream and downstream of foreign DNA of 0.1 kbp to 10 kbp which is then cloned into a plasmid to construct a donor plasmid. As a plasmid vector to be used, any plasmid vector that can be introduced into an animal cell of interest may be used. Examples of such a plasmid vector include pBluescript and pUC57.

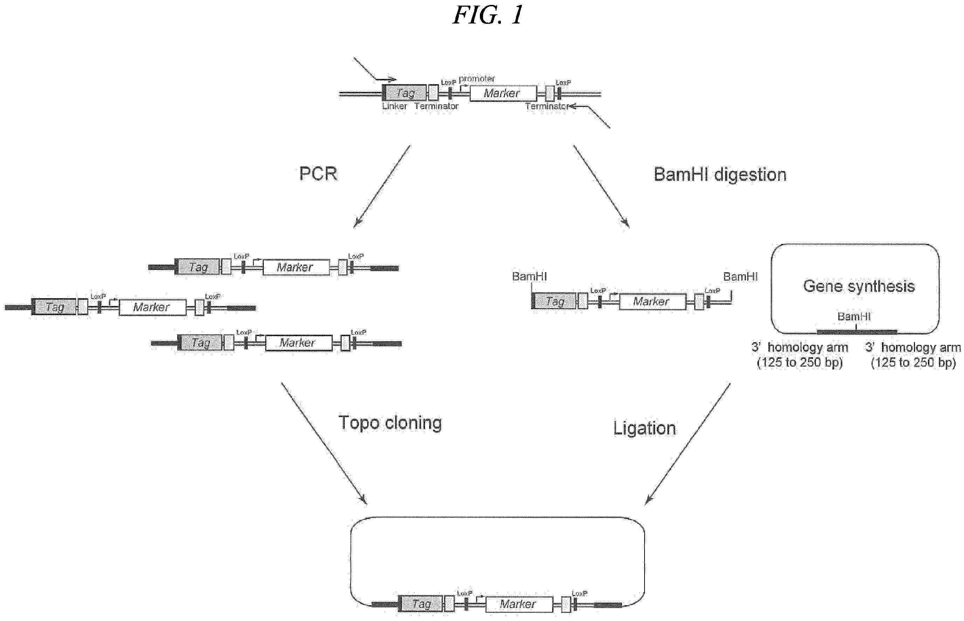

An example of the strategy of step (1) is shown in FIG. 1. As a feature of the present disclosure, there can be mentioned an example in which a donor plasmid can be constructed by such a method utilizing PCR or DNA synthesis.

Step (2) is a step of inserting foreign DNA of 0.1 kbp to 10 kbp into the target site of the genome by a genome editing method using the obtained donor plasmid.

The genome editing method may be any genome-editing technology that induces homology-directed repair (HDR). For example, a CRISPR-Cas9 system, a TALEN system, or a Zn finger nuclease system can be used. in the method of the invention, the CRISPR-Cas9 system is used. The TALEN system is a system using a transcription activator-like effector nuclease (TALEN), and the TALEN is a fusion of a customized binding domain and a non-specific FokI nuclease domain. The DNA-binding domain consists of conserved repeats derived from a transcription activator-like effector (TALE) which is a protein secreted by *Xanthomonas proteobacteria* and altering gene transcription of a host plant. In order to insert the plasmid into the target site of the genome by the TALEN system, TALEN for an insertion site in the genome is constructed, and a donor vector with homology around the insertion site is constructed. Co-introduction of these vectors results in cleavage of the target site, followed by introduction of desired foreign DNA through HDR using a donor vector. By configuring in such a way that foreign DNA is made to have a drug selection marker, a cell in which foreign DNA introduction has occurred can be efficiently selected by drug selection.

On the other hand, the CRISPR-Cas9 system is a system which is possessed by bacteria and archaebacteria and utilizes a locus functioning as a type of acquired immunity against nucleic acids (viral DNA, viral RNA, and plasmid DNA) invaded from the outside. In order to insert the plasmid into the genomic target site by the CRISPR-Cas9 system, a CRISPR-Cas9 vector for cleavage of an insertion site in the genome is constructed. For this purpose, an expression vector encoding a guide RNA that recruits CAS9 at the target site and a CAS9 expression vector are required. By co-introducing these vectors together with a donor vector, the target site is cleaved, followed by introduction of desired foreign DNA through HDR using a donor vector. By configuring in such a way that foreign DNA is made to have a drug selection marker, a cell in which foreign DNA introduction has occurred can be efficiently selected by drug selection.

The Zn finger nuclease (ZFN) system is genome editing using an artificial restriction enzyme consisting of a Zn finger domain and a DNA cleavage domain. Although one Zn finger domain recognizes three bases, a DNA-binding protein that recognizes a specific sequence of 9 to 15 bases can be designed by ligating three to five Zn finger domains. By fusion of a FokI nuclease domain to this DNA-binding protein, an artificial nuclease ZFN that cleaves the target site is designed. In order to insert the plasmid into the genomic target site by the ZFN system, ZFN for an insertion site in the genome is constructed, and a donor vector with homology around the insertion site is constructed. Co-introduction of these vectors results in cleavage of the target site, followed by introduction of desired foreign DNA through HDR using a donor vector. By configuring in such a way that foreign DNA is made to have a drug selection marker, a cell in which foreign DNA introduction has occurred can be efficiently selected by drug selection.

According to the method of the present disclosure, site-specific insertion of foreign DNA of 0.1 kbp to 10 kbp into a genome in an animal cell can be efficiently achieved using a donor plasmid constructed by convenient means such as PCR or DNA synthesis technology. Also, in the case where two selection markers are used at the same time, such foreign DNA can be inserted into both alleles at once. Furthermore, a tag can be added not only to the C-terminus but also to the N-terminus. In addition, construction of a knockout cell can be achieved by stable introduction of foreign DNA in a specific genomic site-specific manner and insertion of a drug selection marker into an endogenous gene-coding region.

### <<Cell obtained by using method for site-specific insertion of foreign DNA into a genome in an animal cell >>

With the method for site-specific insertion of foreign DNA into a genome in an animal cell of the present disclosure, the following cell can be constructed.

In addition, the cell shown below may be an animal cell, and examples of an animal cell include the same cell as exemplified in the foregoing section «Method for site-specific insertion of foreign DNA into a genome in an animal cell ».

### <First instance>

In one instance, described herein is a cell having a chromosome containing a gene encoding transport inhibitor response 1 (TIR1) at a safe harbor locus.

As used herein, the term "safe harbor locus" is a gene region which is constitutively and stably expressed, and means a region where life can be maintained even in the case where the gene originally encoded in the region is deleted or altered. In the case where foreign DNA (a gene encoding TIR1 in the present instance) is inserted into the safe harbor locus using a CRISPR system, a PAM sequence may be in the vicinity thereof. Examples of the safe harbor locus include a GTP-binding protein 10 locus, a Rosa 26 locus, a beta-actin locus, and an AAV integration site 1 (AAVS1) locus. For example, foreign DNA (a gene encoding TIR1 in the present instance) may be inserted into the AAVS 1 locus.

As used herein, the term "TIR1" is an F-box protein which is one of the subunits forming the E3 ubiquitination enzyme complex (SCF complex) in ubiquitin/proteasome-mediated proteolysis and is a plant-specific protein. TIR1 is a receptor for auxin, which is a growth hormone, and it is known that TIR1 accepts auxin to thereby recognize and degrade an inhibitory factor Aux/IAA family protein of the auxin signaling system.

The gene encoding TIR1 is not limited as long as it is a gene encoding TIR1 derived from a plant. Further, the plant from which TIR1 is derived is not limited, and examples thereof include *Arabidopsis thaliana,* rice, common zinnia, pine, fern, and *Physcomitrella patens.* Specific examples of the gene encoding TIR1 include a TIR1 gene, an AFB1 gene, an AFB2 gene, an AFB3 gene, an FBX14 gene, and an AFB5 gene.

The cell of the present instance may have any one type of a gene encoding TIR1 or may have two or more types of a gene encoding TIR1. For example, the sequences of the genes encoding TIR1 derived from *Arabidopsis thaliana* are registered in the TAIR website (http://www.arabidopsis.org/), and Accession No. of each gene is as shown in Table 1 below.

**[Table 1]**

| | TAIR Accession No. |
|---|---|
| TIR1 | AT3G62980.1 |
| AFB1 | AT4G03190.1 |
| AFB2 | AT3G26810.1 |
| AFB3 | AT1G12820.1 |
| FBX14 | AT4G24390.1 |
| AFB5 | AT5G49980.1 |

The gene encoding TIR1 may be, for example, natural DNA extracted from a plant or DNA synthesized by genetic engineering. Further, the gene encoding TIR1 may be, for example, DNA containing exons and introns, or cDNA consisting of exons. The gene encoding TIR1 may be, for example, a full-length genomic DNA sequence or a full-length cDNA sequence. In addition, the gene encoding TIR1 may be a partial genomic DNA sequence or a partial cDNA sequence as long as the expressed protein thereof functions as TIR1.

As used herein, the phrase "functions as TIR1" means, for example, to recognize an Aux/IAA family protein in the presence of auxins. This is because that once TIR1 can recognize the Aux/IAA family protein, it can degrade an Aux/IAA family protein-labeled protein of interest.

### [Promoter]

In the cell of the present instance, a promoter sequence that controls transcription of a gene encoding TIR1 may be operably linked. As a result, TIR1 can be more reliably expressed.

As used herein, the phrase "operably linked" refers to a functional linkage between a gene expression control sequence (for example, a promoter or a series of transcription factor binding sites) and a gene to be expressed (a gene encoding TIR1 in the present instance). As used herein, the term "expression control sequence" refers to a sequence that directs transcription of a gene to be expressed (a gene encoding TIR1 in the present instance).

The promoter is not particularly limited and may be appropriately determined depending on, for example, cell type. Specific examples of the promoter include an inducible promoter, a viral promoter, a housekeeping gene promoter, and a tissue-specific promoter. In the cell of the present instance, the promoter operably linked to the gene encoding TIR1 may be an inducible promoter among those promoters.

### (Inducible promoter)

The inducible promoter is not particularly limited, and examples thereof include a chemically-inducible promoter, a heat shock-inducible promoter, an electromagnetically-inducible promoter, a nuclear receptor-inducible promoter, and a hormone-inducible promoter. In the cell of the present instance, the inducible promoter operably linked to the gene encoding TIR1 may be a chemically-inducible promoter among those promoters.

The chemically-inducible promoter is not particularly limited, and examples thereof include a salicylic acid-inducible promoter (see PCT International Publication No. WO95/19433), a tetracycline-inducible promoter (Gatz et al., (1992) Plant J. 2, pp. 397-404), an ethanol-inducible promoter, and a zinc-inducible metallothionein promoter. In the cell of the present instance, the chemically-inducible promoter operably linked to the gene encoding TIR1 may be a tetracycline-inducible promoter among those promoters.

The heat shock-inducible promoter is not particularly limited, and examples thereof include a heat-inducible promoter (see United States Patent Application, Publication No. 05187287), and a cold-inducible promoter (see United States Patent Application, Publication No. 05847102).

Examples of the electromagnetically-inducible promoter include an early growth response-1 (EGR-1) promoter (see United States Patent Application, Publication No. 5206152) and a c-Jun promoter.

The nuclear receptor-inducible promoter is not particularly limited, and examples thereof include an orphan nuclear receptor chicken ovalbumin upstream promoter.

The hormone-inducible promoter is not particularly limited, and examples thereof include a glucocorticoid-inducible promoter (Lu and Federoff, Hum Gene Ther 6, 419-28, 1995), an MMTV promoter, and a growth hormone promoter.

### (Viral promoter)

The viral promoter is not particularly limited, and examples thereof include a cytomegalovirus (CMV) promoter (CMV immediate early promoter (see, for example, United States Patent No. 5168062), a promoter derived from human immunodeficiency virus (HIV) (for example, a long terminal repeat of HIV), a Rous sarcoma virus (RSV) promoter (for example, a RSV long terminal repeat), a mouse mammary tumor virus (MMTV) promoter, an HSV promoter (for example, a Lap2 promoter or a herpes thymidine kinase promoter, see Wagner et al., Proc. Natl. Acad. Sci., 78, 144-145, 1981.), a promoter derived from SV40 or Epstein Barr virus, and an adeno-associated virus promoter (for example, a p5 promoter).

### (Housekeeping gene promoter)

The housekeeping gene promoter is not particularly limited, and examples thereof include a glyceraldehyde-3-phosphate dehydrogenase (GAPDH) gene promoter, a β-actin gene promoter, a β2-microglobulin gene promoter, and a hypoxanthine phosphoribosyltransferase 1 (HPRT1) gene promoter.

### (Tissue-specific promoter)

The tissue-specific promoter is not particularly limited, and examples thereof include a tyrosinase promoter or TRP2 promoter in the case of melanoma cells or melanocytes; an MMTV promoter or WAP promoter in the case of breast cells or breast cancer; a villin promoter or FABP promoter in the case of intestinal cells or intestinal cancer; a PDX promoter in the case of pancreatic cells; a RIP promoter in the case of pancreatic beta cells; a keratin promoter in the case of keratinocytes; a probasin promoter in the case of prostatic epithelium; a nestin promoter or GFAP promoter in the case of central nervous system (CNS) cells or CNS cancers; a tyrosine hydroxylase, S100 promoter or neurofilament promoter in the case of neurons; a pancreas-specific promoter described in Edlund et al., Science 230:912-916 (1985); a Clara cell secretory protein promoter in the case of lung cancer; and an α-myosin promoter in the case of cardiac cells.

In the present instance, with respect to a gene encoding TIR1, a polyadenylation signal necessary for polyadenylation of the 3' end of mRNA may be operably linked downstream (3' side) of the gene encoding TIR1. The polyadenylation signal may be a polyadenylation signal contained in each of genes derived from the above-mentioned viruses or various human or non-human animals, for example, a polyadenylation signal such as an SV40 late gene or early gene, a rabbit β-globin gene, a bovine growth hormone gene, or a human A3 adenosine receptor gene. In order to further express the gene encoding TIR1, a splicing signal, an enhancer region, or a part of an intron of each gene may be ligated to the 5' upstream of the promoter region, between the promoter region and the translation region, or 3' downstream of the translation region.

In the present instance, with respect to a gene encoding TIR1, one or a plurality of nuclear localization sequences (NLS) may be operably linked upstream (5' side) or downstream (3' side) of the gene encoding TIR1.

Examples of NLS include, but are not limited to, NLS of SV40 virus large T antigen having an amino acid sequence PKKKRKV (SEQ ID NO: 1); NLS derived from nucleoplasmin (for example, bipartite NLS of nucleoplasmin having a sequence KRPAATKKAGQAKKKK (SEQ ID NO: 2)); c-myc NLS having an amino acid sequence PAAKRVKLD (SEQ ID NO: 3) or RQRRNELKRSP (SEQ ID NO: 4); hRNPA1 M9 NLS having a sequence NQSSNFGPMKGGNFGGRSSGPYGGGGQYFAKPRNQGGY (SEQ ID NO: 5); a sequence RMRIZFKNKGKDTAELRRRRVEVSVELRKAKKDEQILKRRNV (SEQ ID NO: 6) of an IBB domain derived from importin-alpha; sequences VSRKRPRP (SEQ ID NO: 7) and PPKKARED (SEQ ID NO: 8) of myoma T protein; human p53 sequence PQPKKKPL (SEQ ID NO: 9); a mouse c-abl IV sequence SALIKKKKKMAP (SEQ ID NO: 10); sequences DRLRR (SEQ ID NO: 11) and PKQKKR (SEQ ID NO: 12) of influenza virus NS1; a sequence RKLKKKIKKL (SEQ ID NO: 13) of hepatitis virus delta antigen; a sequence REKKKFLKRR (SEQ ID NO: 14) of mouse Mx1 protein; a sequence KRKGDEVDGVDEVAKKKSKK (SEQ ID NO: 15) of human poly(ADP-ribose) polymerase; and a sequence RKCLQAGMNLEARKTKK (SEQ ID NO: 16) of steroid hormone receptor (human) glucocorticoid.

In the present instance, with respect to a gene encoding TIR1, one or a plurality of marker genes such as fluorescent protein genes may be operably linked upstream (5' side) or downstream (3' side) of the gene encoding TIR1. Examples of the fluorescent protein include a green fluorescent protein (GFP), a yellow fluorescent protein (YFP), a blue fluorescent protein (BFP), a cyan fluorescent protein, and a red fluorescent protein.

In addition, by introducing a vector including a first gene encoding a target protein, a second gene encoding mini-auxin-inducible degron (mAID) ligated to the downstream of the first gene, and a drug selection marker gene ligated to the downstream of the second gene into the cell of the present instance, such a cell can be used as a degradation evaluation system of a target protein.

Details of the mAID will be described in detail in the section <Third instance> described later. In addition, the vector including the first gene, the second gene, and the drug selection marker gene may be an expression vector. The expression vector is not particularly limited, and examples thereof include a plasmid derived from *Escherichia coli* such as pBR322, pBR325, pUC12, or pUC13; a plasmid derived from *Bacillus subtilis* such as pUB 110, pTP5, or pC194; a plasmid derived from yeast such as pSH19 or pSH15; a bacteriophage such as λ phage; a virus such as adenovirus, adeno-associated virus, lentivirus, vaccinia virus, baculovirus, retrovirus, or hepatitis virus; and a vector obtained by modifying these viruses.

In the vector, a promoter, a polyadenylation signal, NLS, a marker gene of a fluorescent protein, or the like may be operably linked to the 5' end or the 3' end of the first gene, the second gene and the drug selection marker gene.

### <Second instance>

In one instance, described herein is a cell having a chromosome into which foreign DNA has been inserted, using DNA of 100 to 300 bp ligated to the upstream and downstream of such foreign DNA and having a sequence homologous to an insertion site on a chromosome.

In the present instance, the "foreign DNA" is not particularly limited, and examples thereof include the same as those exemplified in the section «Method for site-specific insertion of foreign DNA into animal cell genome».

In the present instance, the length (chain length) of DNA having a sequence homologous to an insertion site on a genome is 100 bp to 300 bp, for example 125 bp to 300 bp, 125 bp to 250 bp, 150 bp to 250 bp, or 170 bp to 250 bp.

In the cell of the present instance, the chromosome into which foreign DNA has been inserted is not particularly limited, and may be, for example, the above-mentioned "safe harbor locus". By being inserted into the "safe harbor locus", the protein encoded by the foreign DNA can be stably and constitutively expressed.

Further, in the cell of the present instance, the chromosome may contain a drug selection marker gene ligated to the region sandwiched between the DNAs of 100 to 300 bp, upstream or downstream of the foreign DNA. Examples of the drug selection marker gene include the same as those exemplified in the section «Method for site-specific insertion of foreign DNA into animal cell genome».

### <Third instance>

In one instance, described herein is a cell including a first chromosome containing a first gene encoding a target protein and a second gene encoding mini-auxin-inducible degron (mAID) ligated to the upstream or downstream of the first gene, and a second chromosome containing a gene encoding TIR1 at a safe harbor locus.

In the cell of the present instance, the "target protein" is not particularly limited as long as it is a protein which is desired to induce degradation thereof in the proteolysis evaluation system using the cell of the present instance. The target protein may be a protein encoded by an inserted foreign gene or may be a cellular endogenous protein.

As used herein, the term "mAID" is a protein consisting of a partial sequence of an Aux/IAA family protein and refers to a protein consisting of a sequence made of a region containing at least two Lys residues at the N-terminal side and the C-terminal side of the domain II region of the Aux/IAA family protein or a sequence formed by linking two or more such sequences.

By including the second gene encoding mAID in the cell of the present instance, as compared with the case of using a full-length Aux/IAA family protein, a domain II region, or the like, an ability to induce degradation of a target protein is improved and cell death is suppressed, whereby stable target proteolytic inducibility is obtained.

Generally, the "Aux/IAA family protein" is a protein having a length of about 25 kDa and is a protein having a domain I, a domain II, a domain III, a domain IV, and the like from the N-terminal side. In the case of a domain II alone among them, there is no improvement in an ability to induce degradation of a target protein, and even in the case of the sequence from the N-terminus to the domain II, there is no improvement in an ability to induce degradation of a target protein.

On the contrary, by incorporating a protein including a sequence consisting of a region which contains at least two Lys residues at the N-terminal side and the C-terminal side of a domain II, does not contain a domain I and may contain a part of a domain III, an ability to induce degradation of a target protein is significantly improved. Further, in the case of using a sequence in which two or more such sequences are linked, an ability to induce degradation of a target protein is further improved.

The gene encoding an Aux/IAA family protein is not particularly limited as long as it is a plant-derived Aux/IAA family gene. The plant is not particularly limited, and the gene may be an *Arabidopsis thaliana* IAA17 gene. Specific examples of the gene encoding an Aux/IAA family protein include an IAA1 gene, an IAA2 gene, an IAA3 gene, an IAA4 gene, an IAA5 gene, an IAA6 gene, an IAA7 gene, an IAA8 gene, an IAA9 gene, an IAA10 gene, an IAA11 gene, an IAA12 gene, an IAA13 gene, an IAA14 gene, an IAA15 gene, an IAA16 gene, an IAA17 gene, an IAA18 gene, an IAA19 gene, an IAA20 gene, an IAA26 gene, an IAA27 gene, an IAA28 gene, an IAA29 gene, an IAA30 gene, an IAA31 gene, an IAA32 gene, an IAA33 gene, and an IAA34 gene.

The cell of the present instance may have a partial sequence of any one of the foregoing genes encoding an Aux/IAA family protein, or may have two or more genes encoding an Aux/IAA family protein. For example, the sequences of the Aux/IAA family genes derived from *Arabidopsis thaliana* are registered in the Arabidopsis Information Resource (TAIR), and Accession No. of each gene is shown below:
IAA1 gene (AT4G14560), IAA2 gene (AT3G23030), IAA3 gene (AT1G04240), IAA4 gene (AT5G43700), IAA5 gene (AT1G15580), IAA6 gene (AT1G52830), IAA7 gene (AT3G23050), IAA8 gene (AT2G22670), IAA9 gene (AT5G65670), IAA10 gene (AT1G04100), IAA11 gene (AT4G28640), IAA12 gene (AT1G04550), IAA13 gene (AT2G33310), IAA14 gene (AT4G14550), IAA15 gene (AT1G80390), IAA16 gene (AT3G04730), IAA17 gene (AT1G04250), IAA18 gene (AT1G51950), IAA19 gene (AT3G15540), IAA20 gene (AT2G46990), IAA26 gene (AT3G16500), IAA27 gene (AT4G29080), IAA28 gene (AT5G25890), IAA29 gene (AT4G32280), IAA30 gene (AT3G62100), IAA31 gene (AT3G17600), IAA32 gene (AT2G01200), IAA33 gene (AT5G57420), and IAA34 gene (AT1G15050).

In the cell of the present instance, the number of amino acids constituting the mAID may be 32 to 80 amino acid residues, for example 50 to 80 amino acid residues, 50 to 75 amino acid residues, or 50 to 70 amino acid residues.

The amino acid sequence constituting the mAID may be a sequence consisting of 32 to 80 amino acid residues, containing 2 to 5 Lys residues or for example 2 to 4 Lys residues at the N-terminal side and the C-terminal side of the domain II region of the Aux/IAA family protein.

In addition, in the cell of the present instance, the first chromosome may contain DNA of 100 to 300 bp having a sequence homologous to an insertion site on a chromosome upstream and downstream of the first gene and the second gene. The length (chain length) of the DNA having a sequence homologous to an insertion site on a genome is 100 bp to 300 bp, for example 125 bp to 300 bp, 125 bp to 250 bp, 150 bp to 250 bp, or 170 bp to 250 bp.

In the cell of the present instance, the first chromosome may contain a drug selection marker gene linked to a region sandwiched between the DNAs of 100 to 300 bp, upstream or downstream of the first gene and the second gene. Examples of the drug selection marker gene include the same as those exemplified in the section «Method for site-specific insertion of foreign DNA into animal cell genome».

In the cell of the present instance, examples of the gene encoding TIR1 include the same as those exemplified in the section <First instance>.

In the cell of the present instance, a promoter sequence that controls transcription of a gene encoding TIR1 may be operably linked. As a result, TIR1 can be more reliably expressed.

The promoter is not particularly limited and may be appropriately determined depending on, for example, cell type. Specific examples of the promoter include an inducible promoter, a viral promoter, a housekeeping gene promoter, and a tissue-specific promoter. In the cell of the present instance, the promoter operably linked to the gene encoding TIR1 may be an inducible promoter among those promoters.

The inducible promoter is not particularly limited, and examples thereof include a chemically-inducible promoter, a heat shock-inducible promoter, an electromagnetically-inducible promoter, a nuclear receptor-inducible promoter, and a hormone-inducible promoter. In the cell of the present instance, the inducible promoter operably linked to the gene encoding TIR1 may be a chemically-inducible promoter among those promoters.

The chemically-inducible promoter is not particularly limited, and examples thereof include the same as those exemplified in the section <First instance>. In the cell of the present instance, the chemically-inducible promoter operably linked to the gene encoding TIR1 may be a tetracycline-inducible promoter among those promoters.

In the cell of the present instance, examples of the heat shock-inducible promoter, the electromagnetically-inducible promoter, the nuclear receptor-inducible promoter, the hormone-inducible promoter, the viral promoter, the housekeeping gene promoter, and the tissue-specific promoter include the same as those exemplified in the section <First instance>.

By using the cell of the present instance, it is possible to easily degrade a target protein. As a method for degrading a target protein using the cell of the present instance, first, auxins of the present instance are allowed to act. The target protein is almost completely degraded in 15 to 30 minutes after auxins are allowed to act thereon.

The amount of auxins to be added is not limited and may be appropriately determined depending on, for example, auxins type. As a specific example, auxins are added to a medium in an amount of 1 µM to 1 mM for example 20 µM to 500 µM.

Examples of the auxin include 1-naphthaleneacetic acid (NAA) and indole-3-acetic acid. In addition to these compounds, a group of compounds having the same physiological activities as those of the NAA or the like can be mentioned, and examples thereof include 2,4-dichlorophenoxyacetic acid, 4-chlorophenoxyacetic acid, (2,4,5-trichlorophenoxy)acetic acid, 1-naphthaleneacetamide, 2,4-dichlorophenoxyacetic acid, and 4-parachloroacetic acid. For example, a precursor that will have the physiological activity of auxin by the means of metabolism can also be used. For example, a substance that is converted into a substance having an auxin activity by the action of esterase or β-oxidase in a host cell may be used. Specific examples thereof include indole-3-acetic acid methyl ester and indole-3-butyric acid.

As for the method of adding auxins, for example, addition of the auxins may be carried out on a medium containing the cells of the present instance.

In this way, since the degradation of a target protein can be rapidly induced by the addition of auxins, the influence of the target protein can be examined by comparing with the case where auxins are not added. More specifically, the influence of the target protein can be examined by, for example, a method in which auxins are added to the cells of the present instance to induce the degradation of the expressed target protein, and then the auxins are removed to suppress the degradation of the newly expressed target protein, or a method in which auxins are added to the cells of the present instance to induce the degradation of the expressed target protein, and then an auxin inhibitor is added to suppress the degradation of the newly expressed target protein.

### EXAMPLES

Hereinafter, the present invention will be described in more detail with reference to Examples and Comparative Examples, but the present invention is not limited to these Examples and the like.

### [Example 1] Addition of GFP to MCM8

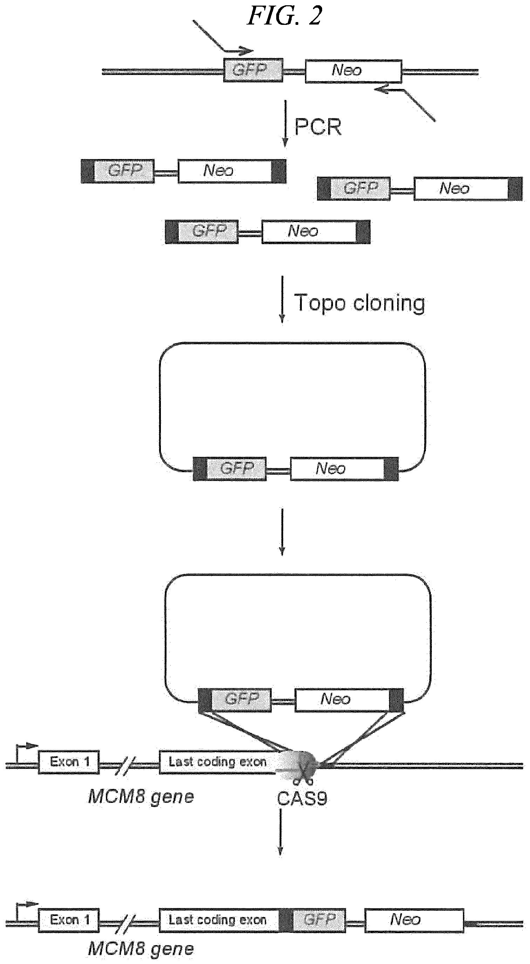

(1) According to the strategy of FIG. 2, a donor plasmid was constructed and a foreign gene was inserted into a target site MCM8 gene on the genome. A GFP-neomycin gene as shown in FIG. 2 was used as the foreign gene.

(2) A pEGFP-C1 vector (available from Clontech Laboratories, Inc.) was used as a template for PCR. In order to amplify the GFP-neomycin gene, two oligo DNAs shown below were synthesized.

In the above SEQ ID NO: 1 and SEQ ID NO: 2, the capital letter part indicates a region of the introduction site (175 b) homologous to the MCM8 genome, and the lower-case letter part indicates a portion (25 b) homologous to pEGFP.

### (3) PCR

The template plasmid and oligo DNAs were PCR-amplified using PrimeSTAR DNA polymerase (available from Takara Bio, Inc.) according to the instruction manual. The amplified DNA was approximately 3.5 kbp.

### (4) Construction of donor plasmid

The PCR-amplified DNA was cloned into a pCR Blunt II-TOPO vector (available from Life Technologies Corporation) by blunt-end ligation according to the instruction manual.

(5) As shown in FIG. 2, the C-terminal coding region of the MCM8 gene was cleaved. In order to give a recognition site to BbsI of a vector pX330 for CRISPR-Cas9 expression, the following oligo DNAs were hybridized and introduced.

5'-caccGCCAGCTTCAAACTATGTAAA-3' (SEQ ID NO: 19)
5'-aaacTTTACATAGTTTGAAGCTGGC-3' (SEQ ID NO: 20)

The construction of pX330 plasmid of CRISPR-Cas9 was carried out according to the method of Nat. Protocols, Vol. 8 No. 11, P2281-2307 (2013).

### (6) Insertion into HCT116 using CRISPR-Cas9

Into proliferating human HCT116 cells in a 6-well plate, 0.8 µg of pX330 for cleaving the target portion of the MCM8 gene and 1 µg of the constructed donor vector were mixed and transfected using Fugene HD (available from Promega Corporation). For transfection, DNA and Fugene HD were mixed according to the instruction manual, allowed to stand for 15 minutes and then added dropwise to the medium.

### (7) Cloning of cells

Two days after the transfection, the cells were diluted and cultured in a medium containing 700 µg/mL of G418 for 13 days, whereby cells that became resistant to neomycin were selected and allowed to form colonies.

### (8) Confirmation of insertion by genomic PCR

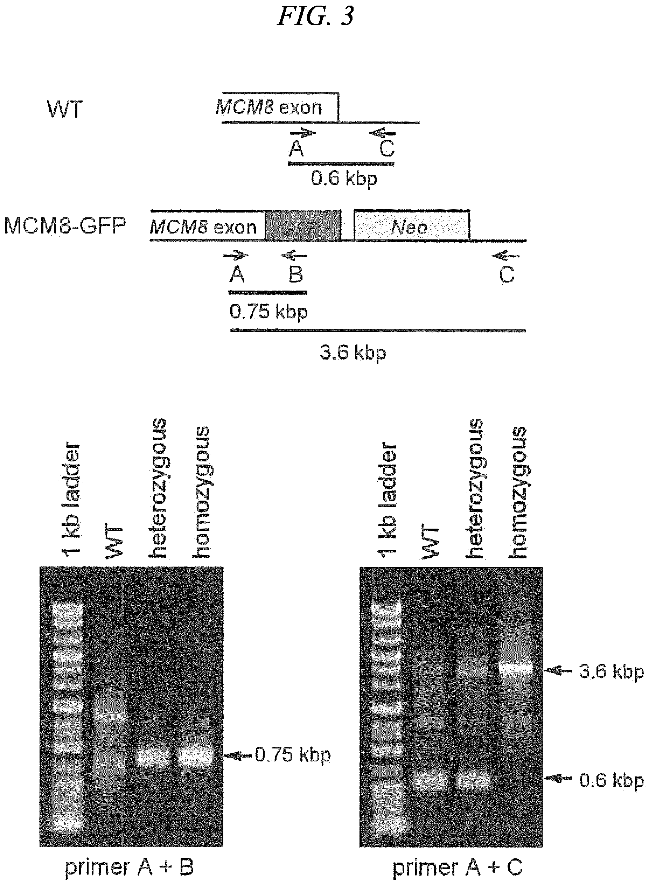

After isolating each colony, genomic DNA was purified after increasing the number of cells, and confirmation of DNA insertion by PCR was carried out. As for the primers used for confirmation of the insertion, a combination of A and B and a combination of A and C shown in FIG. 3 were used. As shown in FIG. 3, in the combination of primers A and B, no specific DNA amplification was observed in the case where the insertion did not occur (WT in left gel photograph). On the other hand, 0.75 kb DNA was amplified in the case in which the expected insertion occurred even in 1 allele (heterozygous and homozygous in left gel photograph). In the combination of primers A and C, 0.6 kb DNA was amplified in the case of an allele where the insertion did not occur, and 3.6 kb DNA was amplified in the case of an allele where the insertion occurred. Both 0.6 kbp and 3.6 kbp DNAs were amplified from clones in which the insertion occurred only in either allele (heterozygous in right gel photograph). In the case where the insertion occurred in both alleles, only 3.6 kbp DNA was amplified (homozygous in right gel photograph).

### (9) Confirmation of expression of MCM8-GFP fusion protein by Western blotting

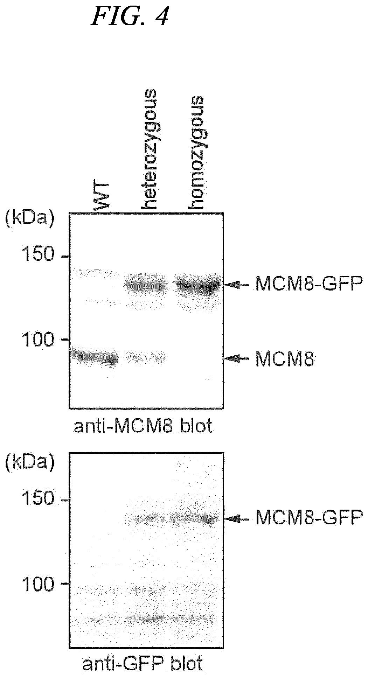

A protein was extracted from clones in which MCM8-GFP insertion was confirmed by genomic PCR and subjected to Western blotting using an MCM8 antibody and a GFP antibody. The MCM8 to which GFP was not added was detected at 93 kDa. Meanwhile, the MCM8-GFP fusion protein was detected at 130 kDa. As shown in FIG. 4, in the case where an MCM8 antibody was used, a 93 kDa band was detected in the original HCT116 cell (WT), whereas in the case where GFP was added to either allele or both alleles, a 140 kDa band was detected. The GFP antibody detects only a 140 kDa fusion protein. From these results, it was confirmed that expected GFP addition to the MCM8 protein occurred.

(9) The relationship between the length of the homologous sequence used and the insertion efficiency is shown in Table 2.

**[Table 2]**

| Length of homologous site | Donor | Number of clones analyzed | Hetero-insertion | Homo-insertion | Insertion efficiency |
|---|---|---|---|---|---|
| 175 bp w/o CRISPR^{∗} | GFP-Neo | 33 | 0 | 0 | 0% |
| 175 bp (exp. 1)^{∗} | GFP-Neo | 28 | 3 | 1 | 14% |
| 175 bp (exp2) ^{∗} | GFP-Neo | 33 | 8 | 0 | 24% |
| 150 bp^{∗} | GFP-Neo | 34 | 3 | 1 | 12% |
| 125 bp^{∗} | GFP-Neo | 36 | 8 | 0 | 22% |
| 80 bp (exp. 1)^{∗} | GFP-Neo | 26 | 0 | 0 | 0% |
| 80 bp (exp. 2)^{∗} | GFP-Neo | 35 | 1 | 0 | 3% |
| 926 bp (L), 740 bp (R)^{∗∗} | mCherry-Hygro | 36 | 19 | 8 | 75% |
| 936 bp (L), 740 bp (R)^{∗∗} | S·tag-3FLAG-Neo | 27 | 11 | 1 | 44% |

| | | | | | |
|---|---|---|---|---|---|
| ^{∗}: PCR was carried out using oligo DNAs having a homologous sequence, and the PCR product was cloned to thereby construct a donor vector. ^{∗∗}: The sequence around the insertion site from the genome was cloned, and the donor vector was generally constructed by subcloning. | | | | | |

From Table 1, it can be seen that the amount of bases at the homologous site is 125 bp or more in length, which is capable of achieving efficient insertion of foreign DNA. Further, it can be seen that the insertion is dependent on site-specific genomic DNA cleavage since insertion of foreign DNA did not occur at all in the case where a pX330 vector for genomic cleavage of the MCM8 gene was absent.

The primers used for PCR of 175 bp are set forth in SEQ ID NO: 21 (5'-CATATTATTATTTGAGAGATTTATTTTAAAAGTAATATTTTGACATGTTACTTA ATGGGCTAAACCTTTTGATGTTTTCTTCCAGGTTGCTGATTTTGAAAATTTTATT GGATCACTAAATGACCAGGGTTACCTCTTGAAAAAAGGCCCAAAAGTTTACC AGCTTCAAACTATGatggtgagcaagggcgaggagctgt-3') and SEQ ID NO: 22 (5'-TATAATACTTTTGGGACATCATTTTTCAGAGAACAGTATTTGACTGTGTGTG TGTGTGTGTGTGTGTGTGTGTGTGTGTGTGTGTGTGTCTGTCTGTCTGTGCGTG CACGCATATCTTCACTGAGATGGCTTTAATCTGCAATAAACCCAGGAGGCCCTA ACTTGGTGAAGTCCTaacgacccaacaccgtgcgttttat-3'), the primers used for PCR of 150 bp are set forth in SEQ ID NO: 23 (5' -TTAAAAGTAATATTTTGACATGTTACTTAATGGGCTAAACCTTTTGATGTTTT CTTCCAGGTTGCTGATTTTGAAAATTTTATTGGATCACTAAATGACCAGGGTTA CCTCTTGAAAAAAGGCCCAAAAGTTTACCAGCTTCAAACTATGatggtgagcaagggc gaggagctgt-3') and SEQ ID NO: 24 (5' -TCAGAGAACAGTATTTGACTGTGTGTGTGTGTGTGTGTGTGTGTGTGTGTG TGTGTGTGTGTGTCTGTCTGTCTGTGCGTGCACGCATATCTTCACTGAGATGGC TTTAATCTGCAATAAACCCAGGAGGCCCTAACTTGGTGAAGTCCTaacgacccaacac cgtgcgttttat-3'), the primers used for PCR of 125 bp are set forth in SEQ ID NO: 25 (5'-CTTAATGGGCTAAACCTTTTGATGTTTTCTTCCAGGTTGCTGATTTTGAAAA TTTTATTGGATCACTAAATGACCAGGGTTACCTCTTGAAAAAAGGCCCAAAAG TTTACCAGCTTCAAACTATGatggtgagcaagggcgaggagctgt-3') and SEQ ID NO: 26 (5' -TGTGTGTGTGTGTGTGTGTGTGTGTGTGTGTGTGTGTGTCTGTCTGTCTGT GCGTGCACGCATATCTTCACTGAGATGGCTTTAATCTGCAATAAACCCAGGAG GCCCTAACTTGGTGAAGTCCTaacgacccaacaccgtgcgttttat-3'), and the primers used for PCR of 80 bp are set forth in SEQ ID NO: 27 (5' -TTGAAAATTTTATTGGATCACTAAATGACCAGGGTTACCTCTTGAAAAAAG GCCCAAAAGTTTACCAGCTTCAAACTATGatggtgagcaagggcgagga-3') and SEQ ID NO: 28 (5'-GTCTGTGCGTGCACGCATATCTTCACTGAGATGGCTTTAATCTGCAATAAAC CCAGGAGGCCCTAACTTGGTGAAGTCCTaacgacccaacaccgtgcgt-3'). In all primers, the capital letter part indicates a region homologous to the surrounding genome of the insertion site, and the lower-case letter part indicates a region homologous to the PCR template.

### [Example 2] Construction of RAD21 degron cells by double knock-in

### (1) Construction of donor vector

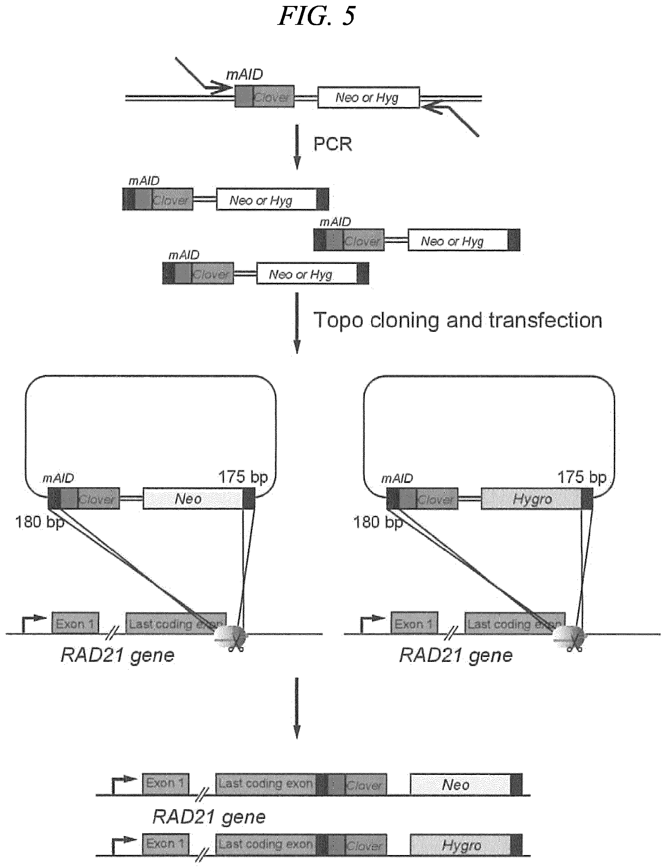

As shown in FIG. 5, a plasmid having mAID (mini AID tag), Clover (modified GFP) and a neomycin- or hygromycin-resistant gene as a template was PCR-amplified with oligo DNA having a homologous region of 180 b (5'-GCTAAAACTGGAGCTGAATCTATCAGTTTGCTTGAGTTATGTCGAAATACG AACAGAAAACAAGCTGCCGCAAAGTTCTACAGCTTCTTGGTTCTTAAAAAGC AGCAAGCTATTGAGCTGACACAGGAAGAACCGTACAGTGACATCATCGCAAC ACCTGGACCAAGGTTCCATATTATAggatccggtgcaggcgcc-3': SEQ ID NO: 29) at 5' and a homologous region of 175 b (5'-TCCCTCAAAGATGAAATTGACAAATTTAATGTACTGGAAAAAAATGAAGA AGGAAAAAGGCAAAGACTTTGTACAGACAAAAATCTAAGTTTTCTCAAAGGG TTCTGTGTCCCCTACACATGGGGGCAATTTGTAAGCACTAGTGAATCAAACAC TAGCTATAATGCTTCTAGCTgcgcgcaattaaccctcactaaagg-3': SEQ ID NO: 30) at 3' to the C-terminal coding region of the RAD21 gene as a primer (a capital letter part indicates a homologous region to RAD21, and a lower-case letter part indicates a homologous region to template DNA) using PrimeSTAR DNA polymerase (available from Takara Bio, Inc.) according to the instruction manual. The amplified DNA was approximately 3.7 kbp. The PCR-amplified DNA was cloned by blunt-end ligation into a pCR Blunt II-TOPO vector (available from Life Technologies Corporation) according to the instruction manual to construct a donor vector.

### (2) Design of RAD21 gene-cleaving CRISPR-Cas9

As shown in FIG. 5, the C-terminal coding region of RAD21 gene was cleaved. In order to give a recognition site to BbsI of a vector pX330 for CRISPR-Cas9 expression, the following oligo DNAs were hybridized and introduced.

5'-caccgCCAAGGTTCCATATTATATA-3' (SEQ ID NO: 31)
5'-aaacTATATAATATGGAACCTTGGc-3' (SEQ ID NO: 32)

The construction of the pX330 plasmid of CRISPR-Cas9 was carried out according to the method of Nat. Protocols, Vol. 8 No. 11, P2281-2307 (2013).

### (3) Insertion into HCT116 using CRISPR-Cas9

In this experiment, wild-type HCT116 cells and HCT 116 cells modified to constitutively express OsTIR1 necessary for an auxin-inducible degron (AID) method were used as materials. Proliferating cells were prepared in a 6-well plate. 0.8 µg of pX330 for cleaving the target portion of RAD21 gene and 0.6 µg of each of the constructed donor vectors having neomycin and hygromycin resistance markers were mixed and transfected into the cells using Fugene HD (available from Promega Corporation). For transfection, DNA and Fugene HD were mixed according to the instruction manual, allowed to stand for 15 minutes and then added dropwise to the medium.

### (4) Cloning of cells

Two days after the transfection, the cells were diluted and cultured in a medium containing 700 µg/mL of G418 and 100 µg/mL of hygromycin for 13 days, whereby cells that became resistant to both neomycin and hygromycin were selected and allowed to form colonies.

### (5) Confirmation of insertion by genomic PCR

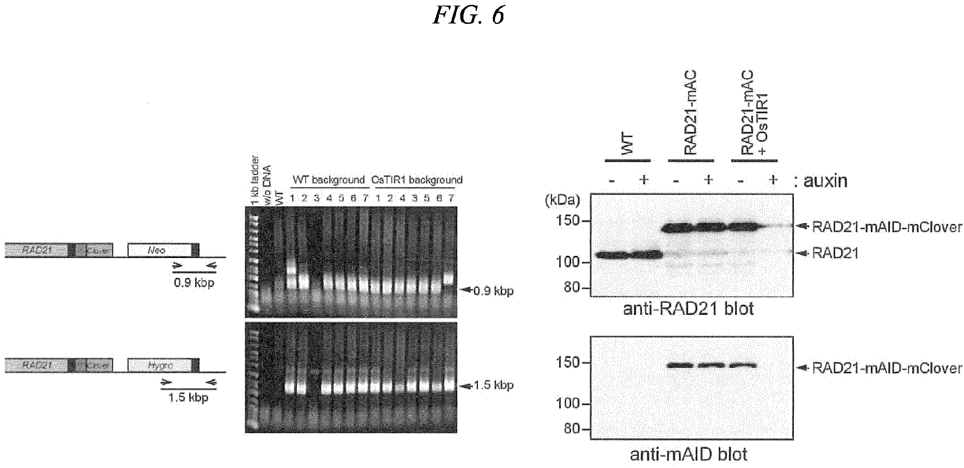

After isolating each colony, genomic DNA was purified after increasing the number of cells, and confirmation of DNA insertion by PCR was carried out. As primers used for the confirmation of insertion, each combination for detecting a neomycin gene and a hygromycin gene shown in FIG. 6 was used. As shown in the gel photograph on the left of FIG. 6, no specific DNA amplification was observed in the case where insertion did not occur (WT in left gel photograph). On the other hand, a 0.9 kbp product was amplified from the neomycin-resistant gene in which the expected insertion occurred, and a 1.5 kbp product was amplified from the hygromycin-resistant gene in which the expected insertion occurred. Both PCR products were detected from many clones (each clone in left gel photograph of FIG. 6). This indicated that a tagging construct having respective resistance markers in both alleles was inserted.

### (6) Confirmation of expression of RAD21-mAID-Clover fusion protein by Western blotting

A protein was extracted from clones in which insertions into both alleles were confirmed by genomic PCR, and subjected to Western blotting using a RAD21 antibody and a mAID antibody (right in FIG. 6). In addition, a protein was similarly extracted from the cells treated with 500 µM of auxin (3-indole acetic acid) for 20 hours and used for Western blotting. The RAD21 to which mAID-Clover was not added was detected at 105 kDa. On the other hand, the RAD21-mAID-Clover fusion protein was detected at 145 kDa. As shown in the right side of FIG. 6, in the case where a RAD21 antibody was used, a 105 kDa band was detected in the original HCT116 cells (WT), whereas in the case where mAID-Clover was added to both alleles, a 145 kDa band was detected. In addition, in the case where a mAID antibody was used, only the RAD21 protein having mAID-Clover added thereto was detected. Further, in the case where cells were treated with auxin, the disappearance of RAD21-mAID-Clover occurred in OsTIR1-expressing cells, indicating that proteolysis by an AID method has occurred.

### (7) Observation of expression of RAD21-mAID-Clover fusion protein by fluorescence microscope

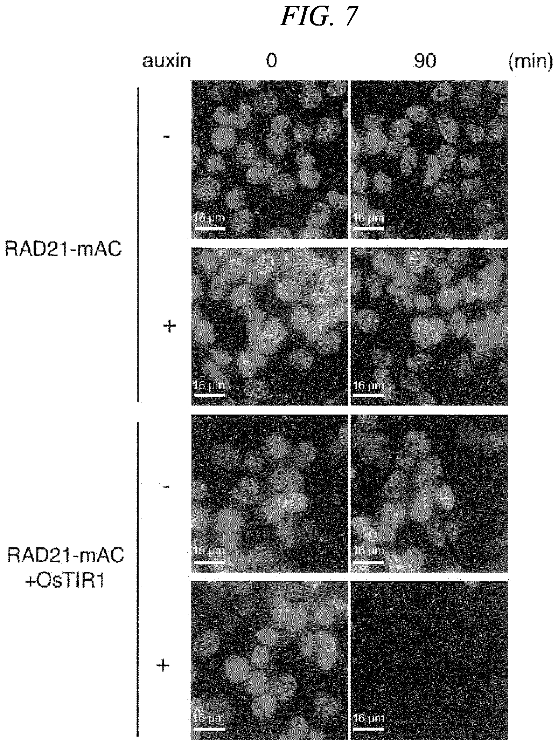

As shown in FIG. 7, living cells in which the RAD21-mAID-Clover fusion protein is expressed were observed under a deconvolution fluorescence microscope. In the cells before treatment with auxin (3-indole acetic acid), it was observed that RAD21-mAID-Clover was localized in the cell nucleus, regardless of expression of OsTIR1. In the case where the cells were treated with 500 µM of auxin and observed after 90 minutes, RAD21-mAID-Clover disappeared only in cells expressing OsTIR1. Also from this result, it can be seen that degradation of RAD21-mAID-Clover by an AID method has occurred.

### [Example 3] Construction of dynein heavy chain 1 (DHC1) degron cells by double knock-in

### (1) Construction of donor vector

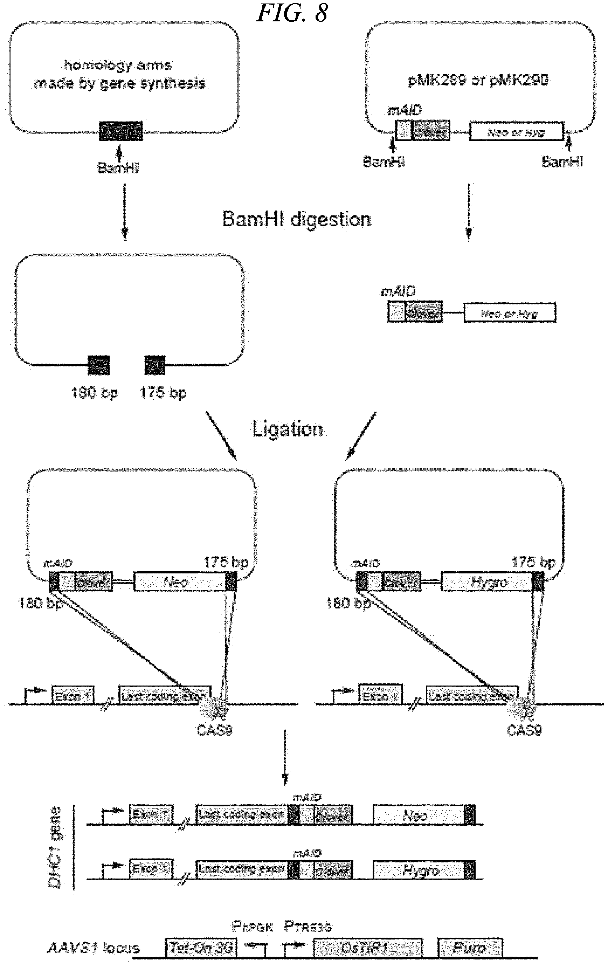

As shown in FIG. 8, a plasmid having mAID (mini AID tag), Clover (modified GFP) and a neomycin- or hygromycin-resistant gene was treated with a restriction enzyme BamHI to obtain a construct consisting of mAID (mini AID tag), Clover (modified GFP) and a neomycin- or hygromycin-resistant gene. Subsequently, homology arms of the genetically synthesized DHC1 gene were constructed by gene synthesis and cloned into the EcoRVs site of pUC57 to obtain a construct (available from GENEWIZ, Inc.). Since the BamHI site has been previously introduced between the homology arms, the plasmid was treated with a restriction enzyme BamHI, and the construct consisting of mAID (mini AID tag), Clover (modified GFP) and a neomycin- or hygromycin-resistant gene was inserted and ligated thereto to construct a donor vector. The resulting donor vector had a region of 180 b (5'-TCCAGTTTTCTTACTTTTCCCTTAAGCCACCAGTAAACCCCTCTGCTTCTGC AGGTAACCTTACCTGTCTACCTGAACTTCACCCGTGCAGACCTCATCTTCACC GTGGACTTCGAAATTGCTACAAAGGAGGATCCTCGCAGCTTCTATGAAAGGGG TGTCGCAGTCTTGTGCACAGAG-3': SEQ ID NO: 33) at 5' and a region of 175 b (5'-ACCACTCCCAACCGTCAGATTCCATTCAGCTTCCTCCAACCTCAGACCAAC CACTTTCTTTTCACAAGCTCAGACCTTCCAAATATTTTAAAAATGAATAAATGT TAAATACCAACTTTCACTATTACAGAAAGGGGCAGCTAGAAAAGTTTACTCTG TGCACAAGACTGCGACA-3': SEQ ID NO: 34) at 3', which are homologous to the DHC1 gene.

### (2) Design of DHC1 gene-cleaving CRISPR-Cas9

As shown in FIG. 8, the C-terminal coding region of the DHC1 gene was cleaved. In order to give a recognition site to BbsI of a vector pX330 for CRISPR-Cas9 expression, the following oligo DNAs were hybridized and introduced.

5'-caccgCCTCGCAGCTTCTACGAGCGGGG-3' (SEQ ID NO: 35)
5'-aaacCCCCGCTCGTAGAAGCTGCGAGGc-3' (SEQ ID NO: 36)

The construction of the pX330 plasmid of CRISPR-Cas9 was carried out according to the method of Nat. Protocols, Vol. 8 No. 11, P2281-2307 (2013).

### (3) Insertion into HCT116 using CRISPR-Cas9

In this experiment, wild-type HCT116 cells and HCT 116 cells modified to constitutively express OsTIR1 necessary for an auxin-inducible degron (AID) method were used as materials. Proliferating cells were prepared in a 6-well plate. 0.8 µg of pX330 for cleaving the target portion of DHC1 gene and 0.6 µg of each of the constructed donor vectors having neomycin and hygromycin resistance markers were mixed and transfected into the cells using Fugene HD (available from Promega Corporation). For transfection, DNA and Fugene HD were mixed according to the instruction manual, allowed to stand for 15 minutes and then added dropwise to the medium.

### (4) Cloning of cells

Two days after the transfection, the cells were diluted and cultured in a medium containing 700 µg/mL of G418 and 100 µg/mL of hygromycin for 13 days, whereby cells that became resistant to both neomycin and hygromycin were selected and allowed to form colonies.

### (5) Confirmation of insertion by genomic PCR

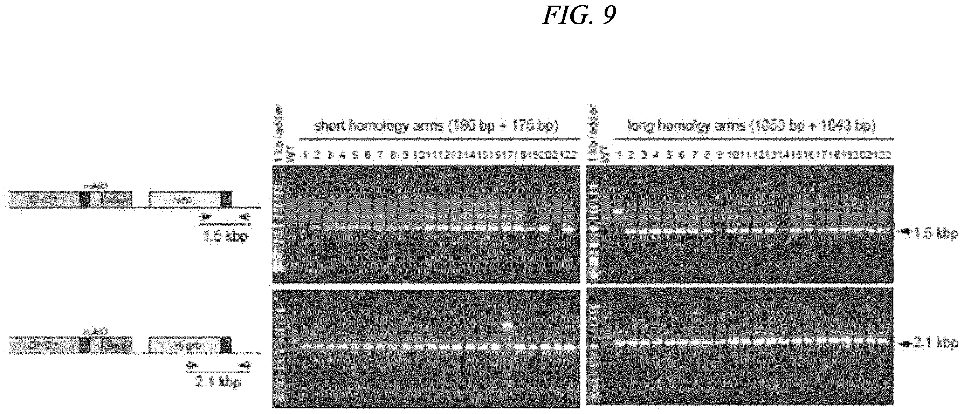

After isolating each colony, genomic DNA was purified after increasing the number of cells, and confirmation of DNA insertion by PCR was carried out. As primers used for the confirmation of insertion, each combination for detecting a neomycin gene and a hygromycin gene shown in FIG. 9 was used. As shown in the gel photograph of FIG. 9, no specific DNA amplification was observed in the case where insertion did not occur (WT in left gel photograph). On the other hand, a 0.9 kbp product was amplified from the neomycin-resistant gene in which the expected insertion occurred, and a 1.5 kbp product was amplified from the hygromycin-resistant gene in which the expected insertion occurred. Both PCR products were detected from many clones (each clone in left gel photograph of FIG. 9). This indicated that a tagging construct having respective resistance markers in both alleles was inserted.

### (6) Confirmation of expression of DHC1-mAID-Clover fusion protein by Western blotting

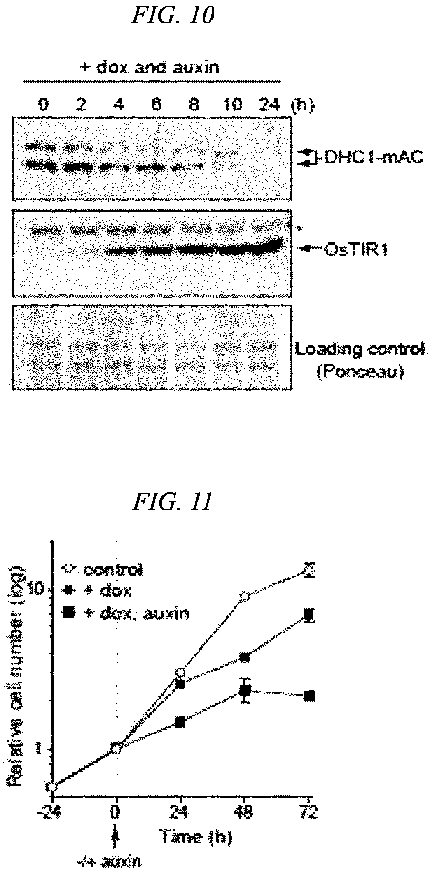

A protein was extracted from clones in which insertions into both alleles were confirmed by genomic PCR, and subjected to Western blotting using a DHC1 antibody and an OsTIR antibody (right in FIG. 10). In addition, a protein was similarly extracted from the cells treated with 2 µg/mL of doxycycline and 500 µM of auxin (3-indole acetic acid) for 2, 4, 6, 8, 10, and 24 hours, and used for Western blotting. As shown in FIG. 10, in the case where a DHC1 antibody was used, the DHC1 protein having mAID-Clover added thereto disappeared with time. On the other hand, in the case where an OsTIR antibody was used, the OsTIR protein increased with time. From these results, it was found that proteolysis by an AID method has occurred.

### (7) Changes in number of cells by treatment with doxycycline and auxin

The relative number of cells at 24, 48, and 72 hours after treatment was calculated with respect to the number of cells at zero hours after treatment, using living cells in which a DHC1-mAID-Clover fusion protein is expressed, untreated and treated with 2 µg/mL of doxycycline alone, or 2 µg/mL of doxycycline and 500 µM of auxin (FIG. 11). As shown in FIG. 11, in the cells treated with 2 µg/mL of doxycycline and 500 µM of auxin, it was found that cell proliferation was suppressed after 48 hours from the treatment.

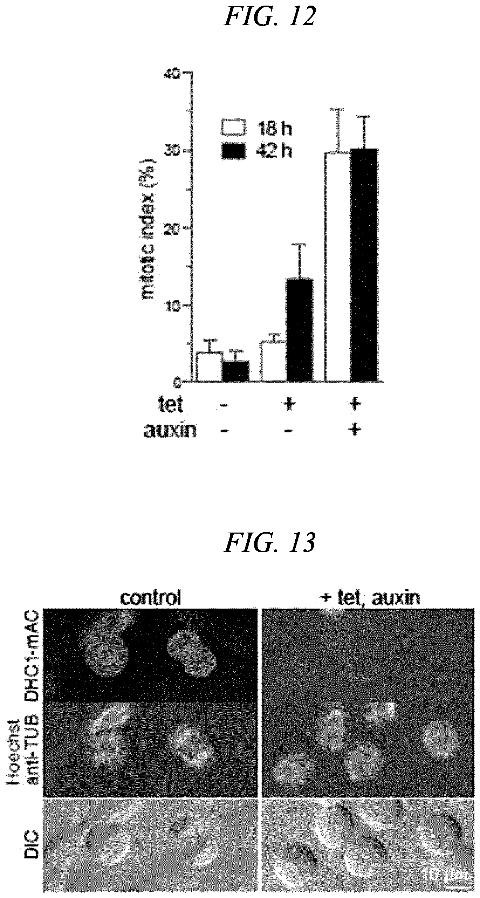

(8) Measurement of percentage of mitotic cells by treatment with tetracycline The percentage of mitotic cells at 18 and 42 hours after treatment was calculated, using living cells in which a DHC1-mAID-Clover fusion protein is expressed, untreated and treated with 1 µg/mL of tetracycline alone, or 1 µg/mL of tetracycline and 500 µM of auxin (FIG. 12).

In addition, fluorescence microscope observation was carried out after immunostaining with a tubulin antibody and nuclear staining with SiR-Hoechst at 18 hours after treatment for living cells in which a DHC1-mAID-Clover fusion protein is expressed, untreated and treated with 1 µg/mL of tetracycline and 500 µM of auxin (FIG. 13).

As shown in FIGS. 12 and 13, it was demonstrated that a chromosomal structural abnormality occurred in the cells treated with 1 µg/mL of tetracycline and 500 µM of auxin, thus resulting in accumulation of mitotic cells.

### [Example 4] Construction of MCM2 degron mouse ES cell by double knock-in

### (1) Construction of donor vector

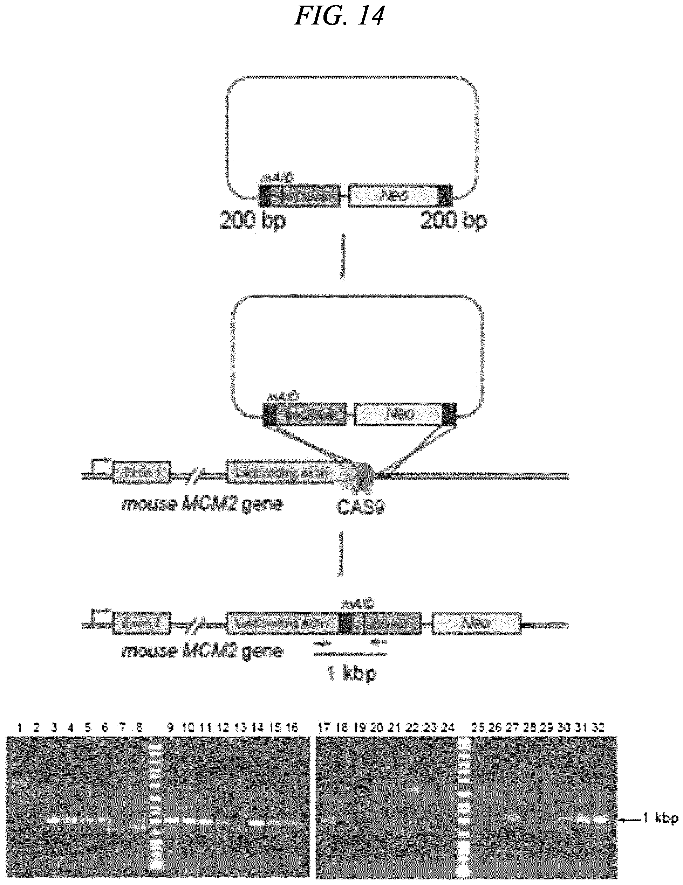

As shown in FIG. 14, a plasmid having mAID (mini AID tag), Clover (modified GFP) and a neomycin-resistant gene was treated with a restriction enzyme BamHI to obtain a construct consisting of mAID (mini AID tag), Clover (modified GFP) and a neomycin-resistant gene. Subsequently, homology arms of the genetically synthesized MCM2 gene were constructed by gene synthesis and cloned into the EcoRVs site of pUC57 to obtain a construct (available from GENEWIZ, Inc.). Since the BamHI site has been previously introduced between the homology arms, the plasmid was treated with a restriction enzyme BamHI, and the construct consisting of mAID (mini AID tag), Clover (modified GFP) and a neomycin- or hygromycin-resistant gene was inserted and ligated thereto to construct a donor vector. The resulting donor vector had a region of 200 b (5'-TCAGGTCTGGAGCTCTGAACCAACACAGCCCTTCCAGAGGTGAAGGCCGG CGTCTTCTCCTGCTAATGGCTCTGATGCTGTTCTTTCTCCAGGCCAGGCAGATC AATATTCACAACCTCTCTGCCTTCTACGACAGCGACCTCTTCAAATTCAACAA GTTCAGCCGTGACCTGAAACGCAAACTGATCCTACAGCAGTTC-3': SEQ ID NO: 37) at 5' and a region of 200 b (5'-ATGAGGCTCGGTCACCACGCTGAGCCTACGTCACTTCCCACTTCCACTGGG GCTTGGTGCCCTGTAGGGGTGGGAGGATGGCTTAATGCAGACCTTTACCTGTG AGCCCCTAGGCCAAGGCTGTAGCATTAAATGACTATTTATTCTTCTGCCCCCCT CTAGAGCACTCTTCTTGGCCAGACCCTCTGTCCAAGGCTCAT-3': SEQ ID NO: 38) at 3', which are homologous to the MCM2 gene.

### (2) Design of MCM2 gene-cleaving CRISPR-Cas9

As shown in FIG. 14, the C-terminal coding region of the MCM2 gene was cleaved. In order to give a recognition site to BbsI of a vector pX330 for CRISPR-Cas9 expression, the following oligo DNAs were hybridized and introduced.

5'-caccgCGAGCCTCATTCAGACATAG-3' (SEQ ID NO: 39)
5'-aaacCTATGTCTGAATGAGGCTCGc-3' (SEQ ID NO: 40)

The construction of the pX330 plasmid of CRISPR-Cas9 was carried out according to the method of Nat. Protocols, Vol. 8 No. 11, P2281-2307 (2013).

### (3) Insertion into HCT116 using CRISPR-Cas9

Mouse ES cells were used as the material in this experiment. Proliferating cells were prepared in a 6-well plate. 0.8 µg of pX330 for cleaving the target portion of MCM2 gene and 0.6 µg of the constructed donor vector having a neomycin resistance marker were mixed and transfected into the cells using Fugene HD (available from Promega Corporation). For transfection, DNA and Fugene HD were mixed according to the instruction manual, allowed to stand for 15 minutes and then added dropwise to the medium.

### (4) Cloning of cells

Two days after the transfection, the cells were diluted and cultured in a medium containing 700 µg/mL of G418 and 100 µg/mL of hygromycin for 13 days, whereby cells that became resistant to neomycin were selected and allowed to form colonies.

### (5) Confirmation of insertion by genomic PCR

After isolating each colony, genomic DNA was purified after increasing the number of cells, and confirmation of DNA insertion by PCR was carried out. As primers used for the confirmation of insertion, a combination for detecting a neomycin gene shown in FIG. 14 was used. As shown in the gel photograph in the lower part of FIG. 14, no specific DNA amplification was observed in the case where insertion did not occur (1, 2, and the like in the lower gel photograph). On the other hand, a 1.0 kbp product was amplified from the neomycin-resistant gene in which the expected insertion occurred. A PCR product was detected from many clones (each clone in the gel photograph in the lower part of FIG. 14). This indicated that a tagging construct having respective resistance markers in both alleles was inserted.

### (6) Observation of expression of MCM2-mAID-Clover fusion protein by fluorescence microscope

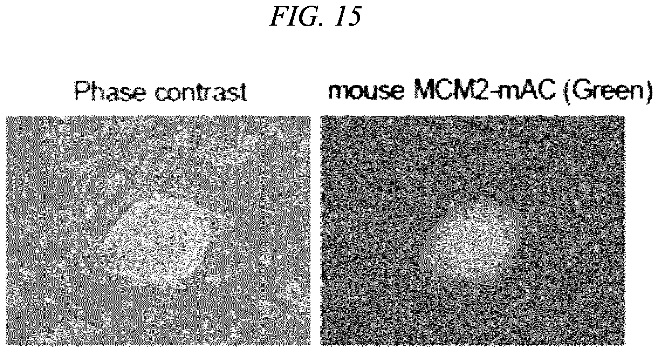

As shown in FIG. 15, mouse ES cells in which the MCM2-mAID-Clover fusion protein is expressed were observed under a deconvolution fluorescence microscope. MCM2-mAID-Clover was observed to be localized in the cell nucleus.

From the above, it was shown that a tagging construct having a resistance marker was inserted in both alleles of mouse ES cells.

### Industrial Applicability

According to the method of the present invention using a donor plasmid, since the length of a sequence homologous to the genomic DNA necessary for HDR using genome editing is as short as 125 bp to 200 bp, there is no need for cloning such a homologous sequence, and such a homologous sequence can be conveniently constructed by PCR or DNA synthesis and is therefore comprehensively applicable to numerous genes and target genomic sites. In addition, a large sequence such as a fluorescent tag, a purification tag, a degron tag, or a drug selection marker may be inserted as the foreign DNA capable of being inserted. In addition, by inserting foreign DNA into any gene by the method of the present invention, it is possible to obtain a cell that constitutively expresses the foreign DNA. For example, according to the cell obtained by the method of the present invention and including a chromosome containing a first gene encoding a target protein and a gene encoding mAID ligated to the downstream of the first gene, and a chromosome containing a gene encoding transport inhibitor response 1 (TIR1) at a safe harbor locus, the degradation of the target protein can be induced efficiently.

## Claims

1. An in vitro method for site-specific insertion of foreign DNA into a genome in an animal cell, comprising:
(1) a step of constructing a donor plasmid in which DNA of 125 bp to 200 bp having a sequence homologous to an insertion site on a genome is ligated to the upstream and downstream of foreign DNA of 0.1 kbp to 10 kbp; and
(2) a step of inserting the foreign DNA of 0.1 kbp to 10 kbp into a target site on the genome by homology-directed repair-based genome editing using the obtained donor plasmid, wherein the animal cell is a human HCT116 cell, or a mouse ES cell,
wherein the homology-directed repair-based genome editing method is a foreign DNA insertion method using a CRISPR-Cas9 system,
and wherein the DNA having a sequence homologous to the inserted genomic site is obtained by PCR or DNA synthesis.

2. The in vitro method for site-specific insertion of foreign DNA into a genome according to Claim 1, wherein the foreign DNA is foreign DNA selected from tagged foreign DNA, a promoter sequence, a transcription termination sequence, a functional gene sequence, a drug selection marker gene, and a combination thereof.

## Patentansprüche

1. In-vitro-Verfahren zur stellenspezifischen Insertion von Fremd-DNA in ein Genom in einer tierischen Zelle, umfassend:
(1) einen Schritt des Konstruierens eines Donorplasmids, in dem DNA von 125 bp bis 200 bp mit einer zu einer Insertionsstelle auf einem Genom homologen Sequenz stromaufwärts und stromabwärts von Fremd-DNA von 0,1 kbp bis 10 kbp ligiert wird; und
(2) einen Schritt des Inserierens der Fremd-DNA von 0,1 kbp bis 10 kbp in eine Zielstelle auf dem Genom durch homologiegerichtete, reparaturbasierte Genom-Editierung unter Verwendung des erhaltenen Donorplasmids, wobei die tierische Zelle eine menschliche HCT116-Zelle oder eine Maus-ES-Zelle ist,
wobei das homologiegerichtete, reparaturbasierte Genome-Editing-Verfahren ein Fremd-DNA-Insertionsverfahren unter Verwendung eines CRISPR-Cas9-Systems ist,
und wobei die DNA mit einer zur inserierten genomischen Stelle homologen Sequenz durch PCR oder DNA-Synthese erhalten wird.

2. In-vitro-Verfahren zur stellenspezifischen Insertion von Fremd-DNA in ein Genom nach Anspruch 1, wobei die Fremd-DNA Fremd-DNA ist, ausgewählt aus markierter Fremd-DNA, einer Promotorsequenz, einer Transkriptionsterminationssequenz, einer funktionellen Gensequenz, einem Wirkstoffauswahl-Markergen und einer Kombination davon.

## Revendications

1. Procédé in vitro permettant l'insertion spécifique à un site d'un ADN étranger dans un génome dans une cellule animale, comprenant :
(1) une étape de construction d'un plasmide donneur dans lequel un ADN de 125 pb à 200 pb présentant une séquence homologue à un site d'insertion sur un génome est ligaturé en amont et en aval d'un ADN étranger de 0,1 kpb à 10 kpb ; et
(2) une étape d'insertion de l'ADN étranger de 0,1 kpb à 10 kpb dans un site cible sur le génome par édition du génome basée sur la réparation dirigée par homologie à l'aide du plasmide donneur obtenu, ladite cellule animale étant une cellule HCT116 humaine ou une cellule ES de souris,
ledit procédé d'édition du génome basée sur la réparation dirigée par homologie étant un procédé d'insertion d'ADN étranger à l'aide d'un système CRISPR-Cas9,
et ledit ADN qui présente une séquence homologue au site génomique inséré étant obtenu par PCR ou synthèse d'ADN.

2. Procédé in vitro permettant l'insertion spécifique à un site d'un ADN étranger dans un génome selon la revendication 1, ledit ADN étranger étant un ADN étranger choisi parmi un ADN étranger marqué, une séquence promoteur, une séquence de terminaison de transcription, une séquence de gène fonctionnel, un gène marqueur de sélection de médicament, et une combinaison de ceux-ci.


### SEQUENCE LISTING

<110> Research Organization of Information and Systems
<120> A technique for site-specific insertion of foreign DNA to the genome of animal cells
<130> PC21721
<160> 40
<170> PatentIn version 3.5
<210> 1
   <211> 7
   <212> PRT
   <213> Artificial Sequence
<220>
   <223> Synthetic DNA
<400> 1
   Pro Lys Lys Lys Arg Lys Val
   1               5
<210> 2
   <211> 16
   <212> PRT
   <213> Artificial Sequence
<220>
   <223> Synthetic DNA
<400> 2
   Lys Arg Pro Ala Ala Thr Lys Lys Ala Gly Gln Ala Lys Lys Lys Lys
   1 5 10 15
<210> 3
   <211> 9
   <212> PRT
   <213> Artificial Sequence
<220>
   <223> Synthetic DNA
<400> 3
   Pro Ala Ala Lys Arg Val Lys Leu Asp
   1 5
<210> 4
   <211> 11
   <212> PRT
   <213> Artificial Sequence
<220>
   <223> Synthetic DNA
<400> 4
   Arg Gln Arg Arg Asn Glu Leu Lys Arg Ser Pro
   1 5 10
<210> 5
   <211> 38
   <212> PRT
   <213> Artificial Sequence
<220>
   <223> Synthetic DNA
<400> 5
   Asn Gln Ser Ser Asn Phe Gly Pro Met Lys Gly Gly Asn Phe Gly Gly
   1 5 10 15
   Arg Ser Ser Gly Pro Tyr Gly Gly Gly Gly Gln Tyr Phe Ala Lys Pro
   20 25 30
   Arg Asn Gln Gly Gly Tyr
   35
<210> 6
   <211> 42
   <212> PRT
   <213> Artificial Sequence
<220>
   <223> Artificial Sequence
<400> 6
   Arg Met Arg Ile Glx Phe Lys Asn Lys Gly Lys Asp Thr Ala Glu Leu
   1               5                   10                  15
   Arg Arg Arg Arg Val Glu Val Ser Val Glu Leu Arg Lys Ala Lys Lys
               20                  25                  30
   Asp Glu Gln Ile Leu Lys Arg Arg Asn Val
           35                  40
<210> 7
   <211> 8
   <212> PRT
   <213> Artificial Sequence
<220>
   <223> Synthetic DNA
<400> 7
   Val Ser Arg Lys Arg Pro Arg Pro
   1               5
<210> 8
   <211> 8
   <212> PRT
   <213> Artificial Sequence
<220>
   <223> Artificial Sequence
<400> 8
   Pro Pro Lys Lys Ala Arg Glu Asp
   1               5
<210> 9
   <211> 8
   <212> PRT
   <213> Artificial Sequence
<220>
   <223> Synthetic DNA
<400> 9
   Pro Gln Pro Lys Lys Lys Pro Leu
   1               5
<210> 10
   <211> 12
   <212> PRT
   <213> Artificial Sequence
<220>
   <223> Synthetic DNA
<400> 10
   Ser Ala Leu Ile Lys Lys Lys Lys Lys Met Ala Pro
   1               5                   10
<210> 11
   <211> 5
   <212> PRT
   <213> Artificial Sequence
<220>
   <223> Synthetic DNA
<400> 11
   Asp Arg Leu Arg Arg
   1               5
<210> 12
   <211> 6
   <212> PRT
   <213> Artificial Sequence
<220>
   <223> Synthetic DNA
<400> 12
   Pro Lys Gln Lys Lys Arg
   1               5
<210> 13
   <211> 10
   <212> PRT
   <213> Artificial Sequence
<220>
   <223> Synthetic DNA
<400> 13
   Arg Lys Leu Lys Lys Lys Ile Lys Lys Leu
   1               5                   10
<210> 14
   <211> 10
   <212> PRT
   <213> Artificial Sequence
<220>
   <223> Synthetic DNA
<400> 14
   Arg Glu Lys Lys Lys Phe Leu Lys Arg Arg
   1               5                   10
<210> 15
   <211> 20
   <212> PRT
   <213> Artificial Sequence
<220>
   <223> Synthetic DNA
<400> 15
   Lys Arg Lys Gly Asp Glu Val Asp Gly Val Asp Glu Val Ala Lys Lys
   1               5                   10                  15
   Lys Ser Lys Lys
               20
<210> 16
   <211> 17
   <212> PRT
   <213> Artificial Sequence
<220>
   <223> Synthetic DNA
<400> 16
   Arg Lys Cys Leu Gln Ala Gly Met Asn Leu Glu Ala Arg Lys Thr Lys
   1               5                   10                  15
   Lys
<210> 17
   <211> 200
   <212> DNA
   <213> Artificial Sequence
<220>
   <223> Synthetic DNA
<400> 17
   catattatta tttgagagat ttattttaaa agtaatattt tgacatgtta cttaatgggc      60
   taaacctttt gatgttttct tccaggttgc tgattttgaa aattttattg gatcactaaa     120
   tgaccagggt tacctcttga aaaaaggccc aaaagtttac cagcttcaaa ctatgatggt     180
   gagcaagggc gaggagctgt                                                 200
<210> 18
   <211> 200
   <212> DNA
   <213> Artificial Sequence
<220>
   <223> Synthetic DNA
<400> 18
   tataatactt ttgggacatc atttttcaga gaacagtatt tgactgtgtg tgtgtgtgtg        60
   tgtgtgtgtg tgtgtgtgtg tgtgtgtgtc tgtctgtctg tgcgtgcacg catatcttca       120
   ctgagatggc tttaatctgc aataaaccca ggaggcccta acttggtgaa gtcctaacga       180
   cccaacaccg tgcgttttat                                                   200
<210> 19
   <211> 25
   <212> DNA
   <213> Artificial Sequence
<220>
   <223> Synthetic DNA
<400> 19
   caccgccagc ttcaaactat gtaaa 25
<210> 20
   <211> 25
   <212> DNA
   <213> Artificial Sequence
<220>
   <223> Synthetic DNA
<400> 20
   aaactttaca tagtttgaag ctggc 25
<210> 21
   <211> 200
   <212> DNA
   <213> Artificial Sequence
<220>
   <223> Synthetic DNA
<400> 21
   catattatta tttgagagat ttattttaaa agtaatattt tgacatgtta cttaatgggc        60
   taaacctttt gatgttttct tccaggttgc tgattttgaa aattttattg gatcactaaa       120
   tgaccagggt tacctcttga aaaaaggccc aaaagtttac cagcttcaaa ctatgatggt       180
   gagcaagggc gaggagctgt                                                   200
<210> 22
   <211> 200
   <212> DNA
   <213> Artificial Sequence
<220>
   <223> Synthetic DNA
<400> 22
   tataatactt ttgggacatc atttttcaga gaacagtatt tgactgtgtg tgtgtgtgtg        60
   tgtgtgtgtg tgtgtgtgtg tgtgtgtgtc tgtctgtctg tgcgtgcacg catatcttca       120
   ctgagatggc tttaatctgc aataaaccca ggaggcccta acttggtgaa gtcctaacga       180
   cccaacaccg tgcgttttat                                                   200
<210> 23
   <211> 175
   <212> DNA
   <213> Artificial Sequence
<220>
   <223> Synthetic DNA
<400> 23
   ttaaaagtaa tattttgaca tgttacttaa tgggctaaac cttttgatgt tttcttccag        60
   gttgctgatt ttgaaaattt tattggatca ctaaatgacc agggttacct cttgaaaaaa       120
   ggcccaaaag tttaccagct tcaaactatg atggtgagca agggcgagga gctgt            175
<210> 24
   <211> 175
   <212> DNA
   <213> Artificial Sequence
<220>
   <223> Synthetic DNA
<400> 24
   tcagagaaca gtatttgact gtgtgtgtgt gtgtgtgtgt gtgtgtgtgt gtgtgtgtgt        60
   gtgtctgtct gtctgtgcgt gcacgcatat cttcactgag atggctttaa tctgcaataa       120
   acccaggagg ccctaacttg gtgaagtcct aacgacccaa caccgtgcgt tttat            175
<210> 25
   <211> 150
   <212> DNA
   <213> Artificial Sequence
<220>
   <223> Synthetic DNA
<400> 25
   cttaatgggc taaacctttt gatgttttct tccaggttgc tgattttgaa aattttattg        60
   gatcactaaa tgaccagggt tacctcttga aaaaaggccc aaaagtttac cagcttcaaa       120
   ctatgatggt gagcaagggc gaggagctgt                                        150
<210> 26
   <211> 150
   <212> DNA
   <213> Artificial Sequence
<220>
   <223> Synthetic DNA
<400> 26
   tgtgtgtgtg tgtgtgtgtg tgtgtgtgtg tgtgtgtgtc tgtctgtctg tgcgtgcacg      60
   catatcttca ctgagatggc tttaatctgc aataaaccca ggaggcccta acttggtgaa     120
   gtcctaacga cccaacaccg tgcgttttat                                      150
<210> 27
   <211> 100
   <212> DNA
   <213> Artificial Sequence
<220>
   <223> Synthetic DNA
<400> 27
   ttgaaaattt tattggatca ctaaatgacc agggttacct cttgaaaaaa ggcccaaaag      60
   tttaccagct tcaaactatg atggtgagca agggcgagga                           100
<210> 28
   <211> 100
   <212> DNA
   <213> Artificial Sequence
<220>
   <223> Synthetic DNA
<400> 28
   gtctgtgcgt gcacgcatat cttcactgag atggctttaa tctgcaataa acccaggagg      60
   ccctaacttg gtgaagtcct aacgacccaa caccgtgcgt                           100
<210> 29
   <211> 198
   <212> DNA
   <213> Artificial Sequence
<220>
   <223> Synthetic DNA
<400> 29
   gctaaaactg gagctgaatc tatcagtttg cttgagttat gtcgaaatac gaacagaaaa 60
   caagctgccg caaagttcta cagcttcttg gttcttaaaa agcagcaagc tattgagctg 120
   acacaggaag aaccgtacag tgacatcatc gcaacacctg gaccaaggtt ccatattata 180
   ggatccggtg caggcgcc 198
<210> 30
   <211> 200
   <212> DNA
   <213> Artificial Sequence
<220>
   <223> Synthetic DNA
<400> 30
   tccctcaaag atgaaattga caaatttaat gtactggaaa aaaatgaaga aggaaaaagg 60
   caaagacttt gtacagacaa aaatctaagt tttctcaaag ggttctgtgt cccctacaca 120
   tgggggcaat ttgtaagcac tagtgaatca aacactagct ataatgcttc tagctgcgcg 180
   caattaaccc tcactaaagg 200
<210> 31
   <211> 25
   <212> DNA
   <213> Artificial Sequence
<220>
   <223> Synthetic DNA
<400> 31
   caccgccaag gttccatatt atata 25
<210> 32
   <211> 25
   <212> DNA
   <213> Artificial Sequence
<220>
   <223> Synthetic DNA
<400> 32
   aaactatata atatggaacc ttggc 25
<210> 33
   <211> 180
   <212> DNA
   <213> Artificial Sequence
<220>
   <223> Synthetic DNA
<400> 33
   tccagttttc ttacttttcc cttaagccac cagtaaaccc ctctgcttct gcaggtaacc 60
   ttacctgtct acctgaactt cacccgtgca gacctcatct tcaccgtgga cttcgaaatt 120
   gctacaaagg aggatcctcg cagcttctat gaaaggggtg tcgcagtctt gtgcacagag 180
<210> 34
   <211> 175
   <212> DNA
   <213> Artificial Sequence
<220>
   <223> Synthetic DNA
<400> 34
   accactccca accgtcagat tccattcagc ttcctccaac ctcagaccaa ccactttctt 60
   ttcacaagct cagaccttcc aaatatttta aaaatgaata aatgttaaat accaactttc 120
   actattacag aaaggggcag ctagaaaagt ttactctgtg cacaagactg cgaca 175
<210> 35
   <211> 28
   <212> DNA
   <213> Artificial Sequence
<220>
   <223> Synthetic DNA
<400> 35
   caccgcctcg cagcttctac gagcgggg 28
<210> 36
   <211> 28
   <212> DNA
   <213> Artificial Sequence
<220>
   <223> Synthetic DNA
<400> 36
   aaacccccgc tcgtagaagc tgcgaggc 28
<210> 37
   <211> 200
   <212> DNA
   <213> Artificial Sequence
<220>
   <223> Synthetic DNA
<400> 37
   tcaggtctgg agctctgaac caacacagcc cttccagagg tgaaggccgg cgtcttctcc 60
   tgctaatggc tctgatgctg ttctttctcc aggccaggca gatcaatatt cacaacctct 120
   ctgccttcta cgacagcgac ctcttcaaat tcaacaagtt cagccgtgac ctgaaacgca 180
   aactgatcct acagcagttc 200
<210> 38
   <211> 200
   <212> DNA
   <213> Artificial Sequence
<220>
   <223> Synthetic DNA
<400> 38
   atgaggctcg gtcaccacgc tgagcctacg tcacttccca cttccactgg ggcttggtgc 60
   cctgtagggg tgggaggatg gcttaatgca gacctttacc tgtgagcccc taggccaagg 120
   ctgtagcatt aaatgactat ttattcttct gcccccctct agagcactct tcttggccag 180
   accctctgtc caaggctcat 200
<210> 39
   <211> 25
   <212> DNA
   <213> Artificial Sequence
<220>
   <223> Synthetic DNA
<400> 39
   caccgcgagc ctcattcaga catag 25
<210> 40
   <211> 25
   <212> DNA
   <213> Artificial Sequence
<220>
   <223> Synthetic DNA
<400> 40
   aaacctatgt ctgaatgagg ctcgc 25